# EUROPEAN PATENT APPLICATION

(11) **EP 4 101 865 A1**
(43) Date of publication of application: **14.12.2022**
(21) Application number: 20918025.6
(22) Date of filing: 27.11.2020
(51) Int. Cl.: C07K 16/28, C12N 15/85, C07K 14/005, A61P 35/00, A61P 31/00, A61K 39/00

(54) **TCR-LIKE ANTIBODY SPECIFIC TO CMV PP65 PEPTIDE/HLA-A*02 COMPLEX, AND USE THEREOF**

(30) Priority: 06.02.2020 KR 20200014469
(71) Applicant: Ajou University Industry-Academic Cooperation Foundation, Gyeonggi-do 16499 (KR)
(72) Inventor: KIM, Yong Sung, Suwon-si Gyeonggi-do 16534 (KR); LEE, Seyoung, Suwon-si Gyeonggi-do 16499 (KR)
(74) Representative: Mewburn Ellis LLP
(86) International application number: PCT/KR2020/017067
(87) International publication number: WO 2021/157830

(57) **Abstract**

The present invention provides a TCR-like antibody or an antigen-binding fragment thereof, the antibody binding to and having specific and improved affinity to an MHC-I molecule, particularly a CMV pp65 peptide complex (CMVP495-503/HLA-A*02:01) presented by HLA-A^{*}02. The present invention also relates to: a nucleic acid for coding the TCR-like antibody or the antigen-binding fragment thereof according to the present invention; an expression vector comprising the nucleic acid; a cell transformed to the expression vector; a method for producing same; a composition comprising a T cell for expressing the antibody or the antigen-binding fragment thereof as a chimeric antigen receptor; a composition for preventing or treating cancer or an infectious disease; and a composition for diagnosis.

## Description

### [Technical Field]

The present invention relates to a TCR (T-cell receptor)-like antibody that binds specifically and with improved affinity to a CMV pp65 peptide complex (CMV_{P}495-503/HLA-A*02:01) presented by an MHC-I molecule, particularly HLA-A*02:01, or an antigen-binding fragment thereof.

The present invention also relates to a nucleic acid encoding the TCR-like antibody according to the present invention or the antigen-binding fragment thereof, an expression vector containing the nucleic acid, a cell transformed with the expression vector, a method of producing the same, and a composition containing T cells expressing the antibody or the antigen-binding fragment thereof as a chimeric antigen receptor, preferably a composition for preventing or treating cancer or an infectious disease and a composition for diagnosing cancer or an infectious disease.

### [Background Art]

A major histocompatibility complex (MHC) is a set of genes encoding cell-surface proteins for recognizing foreign molecules in vertebrates, and acts as an antigen-presenting molecule that recognizes the target material of an immune response as a T-cell antigen. MHC is broadly divided into two types: type-I MHC (MHC class I molecules, MHC-I) and type-II MHC (MHC class II molecules, MHC-II). Human MHC-II is a heterodimer structure composed of an alpha chain (a-chain) and a beta chain (β-chain). Human MHC-I is a heterodimer structure of human leukocyte antigen (HLA) and beta-2-microglobulin (β2m). Human MHC-I has three types of genes, namely HLA-A, HLA-B, and HLA-C, and is expressed in all cells having a nucleus, including tumor cells. Antigens presented by MHC-I react with CD8+ T cells. MHC-II has genes of HLA-DP, HLA-DQ, and HLA-DR, and proteins are expressed in antigen-presenting cells (APCs), such as dendritic cells (DCs), macrophages, and B cells. Antigens presented by MHC-II react with CD4+ T cells.

Tumor- and virus-infected cells may present peptide antigens (8-10 amino acid residues) called peptide/MHC-I complexes via cell-surface MHC-I molecules with respect to CD8+ cytotoxic T lymphocytes (CTLs), and the antigen-derived peptide presented by MHC is referred to as a T-cell epitope. CD8+ T cells are able to recognize an antigen epitope peptide only when an antigen is presented in a form of a peptide bound to MHC-I, namely a peptide/MHC-I complex. This antigen recognition of T cells is called MHC-restricted antigen recognition (MHC restriction). CD8+ cytotoxic T cells enable recognition and binding through a T-cell receptor (TCR) specific to the peptide/MHC-I complex, and secrete effector materials to thus kill target cells. Malignantly transformed or virus-infected cells present tumor-associated antigens or viral protein antigen-derived peptides as peptide/MHC-I complexes via MHC-I molecules. Thus, disease-specific peptide/MHC-I complexes may be the target of immunotherapeutic approaches.

Since the T-cell receptor has inherent specificity to the peptide/MHC complex but has low intrinsic affinity thereto, practical use thereof is difficult. Accordingly, an antibody in which inherent specificity to the peptide/MHC complex is maintained and which has high affinity has been developed, and is called a TCR-like antibody. A TCR-like antibody is being developed as a new type of immunotherapeutic agent capable of targeting tumor- and virus-infected cells and mediating specific death thereof. In addition to therapeutic purposes, a TCR-like antibody is being developed as a diagnostic reagent for cancer and infectious diseases, and is also useful as a tool for researching the MHC-I pathway by visualizing MHC-I antigen presentation.

An MHC-I antigen-presenting molecule is composed of alpha chains (alpha chains 1, 2, 3) and beta-2-microglobulin (β2m), and a total of six different antigen-presenting molecules, including two HLA-As, two HLA-Bs, and two HLA-Cs, are generated depending on the type of alpha chain. Among HLA-A proteins, the HLA-A*02 type accounts for about 25-30% of the global population, and in particular, the HLA-A*02:01 subtype accounts for 30-90% depending on race, and is thus regarded as the most common MHC-I molecule (Lai, Choo et al. 2017).

Human cytomegalovirus (CMV) is a beta-herpes virus that infects humans, monkeys, and rodents and produces unique large cells having intranuclear inclusions. 70% of people are infected with CMV worldwide. Primary infection with CMV persists for life in a latent form, and therefore healthy adults infected therewith are asymptomatic. However, people having weakened immunity, such as immunocompromised organ transplant recipients or people infected with human immunodeficiency virus (HIV), are at high risk of developing life-threatening diseases due to reactivation of CMV.

The immune system against CMV is complicated and is accompanied by humoral and cellular responses. Studies have reported that natural killer cells (NK cells) and CD8+ cytotoxic T cells play an important role in the prevention of CMV recurrence. Although many gene products of CMV induce cytotoxic T-cell responses to CMV infection, the high expression frequency of pp65 (phosphoprotein 65) protein suggests pp65 protein as a major target of cytotoxic T-lymphocyte-mediated immune responses. Among pp65 protein peptides, the CMV-specific cytotoxic T-cell activity of HLA-A*02:01 positive individuals is mainly caused by the pp65 protein amino acid residue sequence 495-503-derived peptide (CMV_{P}495-503, 495-NLVPMVATV-503) (Chee, Bankier et al. 1990). In particular, it has been reported that the frequency of CD8+ T cells that specifically recognize the CMV_{P}495-503/HLA-A*02:01 complex in the blood of healthy CMV-infected persons accounts for 0.5 to 11% of a total of CD8+ T cells (Schmittnaegel, Levitsky et al. 2015)

Based on the results of measurement of affinity of the H9 clone in IgG form, which is a conventionally developed TCR-like antibody specifically binding to the CMV_{P}495-503/HLA-A*02:01 complex, through SPR (surface plasmon resonance), the association rate constant (kₒₙ) was determined to be kₒₙ = 6.5×10⁴ 1/Ms, the dissociation rate constant (k_{off}) was determined to be k_{off} = 0.0207 1/s, and the equilibrium dissociation constant (K_{D}) was determined to be K_{D} = 300 nM (Makler, Oved et al. 2010) . This low affinity makes it difficult to detect a peptide/MHC complex, the expression level of which is very low compared to other cell-surface antigens.

The TCR-like antibody having improved affinity to the CMV_{P}495-503/HLA-A*02:01 complex according to the present invention is capable of detecting the CMV_{P}495-503/HLA-A*02:01 complex, which has been impossible to detect using known conventional antibodies, even in cell lines in which the expression level of the HLA-A*02:01 molecule is low, so utility thereof is high due to the remarkably high affinity thereof.

Accordingly, the TCR-like antibody having improved affinity to the CMV_{P}495-503/HLA-A*02:01 complex according to the present invention is effective at detecting the CMV_{P}495-503/HLA-A*02:01 complex even in an environment in which the CMV_{P}495-503/HLA-A*02:01 complex is presented in a small amount due to reduction or loss of MHC expression, such as a tumor microenvironment.

### [Disclosure]

It is an object of the present invention to provide a TCR-like antibody, which is specific to a CMV_{P}495-503/HLA-A*02:01 complex in which an amino acid 495-503-derived peptide of pp65 protein of human cytomegalovirus (CMV) (CMV_{P}495-503, 495-NLVPMVATV-503) is presented to MHC-I, namely HLA-A*02:01, and which has remarkably improved affinity thereto, or an antigen-binding fragment thereof.

It is another object of the present invention to provide a heavy-chain variable domain (VH) and a light-chain variable domain (VL) of the improved TCR-like antibody.

It is still another object of the present invention to provide complementarity-determining regions (CDRs), which are antigen-binding sites in the heavy-chain variable region and the light-chain variable region of the improved TCR-like antibody.

It is yet another object of the present invention to provide a protein recombinant monoclonal antibody using partial and full amino acid sequences derived from the TCR-like antibody having improved affinity and high specificity or the antigen-binding fragment thereof.

It is still yet another object of the present invention to provide a T-cell receptor, preferably a chimeric antigen receptor (CAR), using partial and full amino acid sequences derived from the TCR-like antibody having improved affinity and high specificity.

It is a further object of the present invention to provide a modified T cell expressing a T-cell receptor CAR using partial and full amino acid sequences derived from the TCR-like antibody having improved affinity and high specificity.

It is still a further object of the present invention to provide a polynucleotide encoding the TCR-like antibody having improved affinity to the CMV_{P}495-503/HLA-A*02 complex.

It is yet a further object of the present invention to provide a cell including the polynucleotide or the expression vector.

It is still yet a further object of the present invention to provide a method for research into presentation of an amino acid residue sequence 495-503-derived peptide of pp65 protein of human cytomegalovirus (CMV) (CMV_{P}495-503, 495-NLVPMVATV-503) to HLA-A*02, which is MHC-I.

It is even still yet a further object of the present invention to provide a composition for diagnosing cancer or an infectious disease resulting from infection with human cytomegalovirus (CMV), or a pharmaceutical composition for preventing or treating the same.

In order to accomplish the above objects, the present invention provides a TCR-like antibody specifically binding to a complex of MHC-I presenting a viral protein antigen-derived peptide or an antigen-binding fragment thereof.

The viral protein antigen-derived peptide may be peptide 495-503 or peptide 480-503 of the pp65 protein of human cytomegalovirus (CMV) of SEQ ID NO: 1 or 2.

MHC-I presenting the viral protein antigen-derived peptide may be HLA-A*02:01.

An aspect of the present invention provides a TCR-like antibody having improved affinity to the CMV_{P}495-503/HLA-A*02:01 complex. This antibody is able to bind with high affinity to CMV_{P}495-503/HLA-A*02:01 molecules compared to conventional antibody fragments known to bind thereto, and is also able to detect the complex of MHC-I presenting the viral protein antigen-derived peptide even in an environment in which MHC-I expression is reduced or lost, such as a tumor microenvironment.

In an embodiment of the present invention, a yeast cell surface scFab (single-chain Fab (antigen-binding fragment)) library was designed and constructed, clones showing higher affinity than conventionally known antibodies to the CMV_{P}495-503/HLA-A*02:01 complex were selected, isolated, and identified, and the variable region sequence of the selected clones was inserted into mouse IgG2a to construct an intact IgG antibody, which was then purified.

In another embodiment of the present invention, a TCR-like antibody in an intact IgG form specific to the CMV_{P}495-503/HLA-A*02:01 complex was quantitatively analyzed for improved affinity to the CMV_{P}495-503/HLA-A*02:01 complex using ELISA and Octet.

In an embodiment of the present invention, the CMV_{P}495-503/HLA-A*02:01 complex was quantitatively analyzed on the cell surface subjected to CMV_{P}495-503 peptide pulsing using the TCR-like antibody in an intact IgG form specific to the CMV_{P}495-503/HLA-A*02:01 complex, and the CMV_{P}495-503/HLA-A*02:01 complex was effectively detected even in cells in which the expression of MHC molecules was relatively low.

In an embodiment of the present invention, it was confirmed that the CMV_{P}495-503/HLA-A*02:01 complex could be detected on the cell surface when cells were treated with a viral epitope fusion antibody (viral antigen-derived T-cell epitope peptide-fused cytotransmab) in which a peptide (CMV_{P}480-503) containing a CMV pp65-derived T-cell epitope is fused to a cytosol-penetrating antibody (cytotransmab), using the TCR-like antibody specific to the CMV_{P}495-503/HLA-A*02:01 complex.

The TCR-like antibody according to the present invention having the characteristics described above or the antigen-binding fragment thereof specifically binds to the complex of MHC-I presenting the viral protein antigen-derived peptide. Here, the viral protein antigen-derived peptide is peptide 495-503 or peptide 480-503 of the pp65 protein of human cytomegalovirus (CMV), and the MHC-I is HLA-A*02:01.

The TCR-like antibody according to the present invention or the antigen-binding fragment thereof includes a heavy-chain CDR1 of SEQ ID NO: 12, a heavy-chain CDR2 selected from the group consisting of SEQ ID NOs: 13, 17, and 18, a heavy-chain CDR3 selected from the group consisting of SEQ ID NOs: 15 and 16, a light-chain CDR1 of SEQ ID NO: 5, a light-chain CDR2 selected from the group consisting of SEQ ID NOs: 6, 10, and 11, and a light-chain CDR3 selected from the group consisting of SEQ ID NOs: 8 and 9.

Specifically, it includes a light-chain CDR1 of SEQ ID NO: 5, a light-chain CDR2 of SEQ ID NO: 6, a light-chain CDR3 of SEQ ID NO: 8, a heavy-chain CDR1 of SEQ ID NO: 12, a heavy-chain CDR2 of SEQ ID NO: 13, and a heavy-chain CDR3 of SEQ ID NO: 15;
a light-chain CDR1 of SEQ ID NO: 5, a light-chain CDR2 of SEQ ID NO: 6, a light-chain CDR3 of SEQ ID NO: 9, a heavy-chain CDR1 of SEQ ID NO: 12, a heavy-chain CDR2 of SEQ ID NO: 13, and a heavy-chain CDR3 of SEQ ID NO: 16;
a light-chain CDR1 of SEQ ID NO: 5, a light-chain CDR2 of SEQ ID NO: 10, a light-chain CDR3 of SEQ ID NO: 9, a heavy-chain CDR1 of SEQ ID NO: 12, a heavy-chain CDR2 of SEQ ID NO: 17, and a heavy-chain CDR3 of SEQ ID NO: 16; or
a light-chain CDR1 of SEQ ID NO: 5, a light-chain CDR2 of SEQ ID NO: 11, a light-chain CDR3 of SEQ ID NO: 9, a heavy-chain CDR1 of SEQ ID NO: 12, a heavy-chain CDR2 of SEQ ID NO: 18, and a heavy-chain CDR3 of SEQ ID NO: 16.

Preferably, the TCR-like antibody (T-cell receptor-like antibody) according to the present invention or the antigen-binding fragment thereof includes a light-chain variable region selected from the group consisting of SEQ ID NOs: 20 to 23 and/or a heavy-chain variable region selected from the group consisting of SEQ ID NOs: 25 to 28, but is not limited thereto.

Specifically, it includes a light-chain variable region of SEQ ID NO: 20 and a heavy-chain variable region of SEQ ID NO: 25;
a light-chain variable region of SEQ ID NO: 21 and a heavy-chain variable region of SEQ ID NO: 26;
a light-chain variable region of SEQ ID NO: 22 and a heavy-chain variable region of SEQ ID NO: 27; or
a light-chain variable region of SEQ ID NO: 23 and a heavy-chain variable region of SEQ ID NO: 28.

In addition, the present invention relates to a nucleic acid encoding the antibody or the antigen-binding fragment thereof and an expression vector including the nucleic acid.

In addition, the present invention relates to a cell transformed with the expression vector.

In addition, the present invention relates to a method of producing a TCR-like antibody or an antigen-binding fragment thereof, including:
(a) culturing the cell described above; and
(b) recovering an antibody or an antigen-binding fragment thereof from the cultured cell.

In addition, the present invention relates to a composition including T cells expressing the antibody or the antigen-binding fragment thereof as a chimeric antigen receptor.

In addition, the present invention relates to a composition for preventing or treating cancer or an infectious disease including the antibody or the antigen-binding fragment thereof.

In addition, the present invention relates to a composition for diagnosing cancer or an infectious disease including the antibody or the antigen-binding fragment thereof.

### [Description of Drawings]

FIG. 1 shows the low affinity and non-specific binding of an H9 clone, which is a conventional TCR-like antibody specific to a CMV_{P}495-503/HLA-A*02:01 complex,
FIG. 1a schematically showing an expression vector for expressing the H9 clone in the form of a full-length IgG antibody, in which the heavy-chain variable region (VH) and the light-chain variable region (VL) of the H9 clone were introduced to the heavy-chain constant region (CH1-hinge-CH2-CH3 domain) of mouse immunoglobulin 2a (mIgG2a) and the mouse kappa light-chain constant region (kappa domain) and expressed in animal cells (HEK293F),
FIG. 1b showing the results of flow cytometry analysis to determine the ability of the H9 antibody to bind to the CMV_{P}495-503/HLA-A*02:01 complex, after pulsing of 50 µM CMV_{P}495-503 peptide in cells having various cell-surface MHC-I (HLA-A*02:01) expression levels through expression and purification of the constructed H9 in a full-length IgG form,
FIG. 1c showing the results of flow cytometry analysis to determine the ability of the H9 antibody to bind to the CMV_{P}495-503/HLA-A*02:01 complex, after inducing the formation of the CMV_{P}495-503/HLA-A*02:01 complex by treating MDA-MB-231 cells with viral epitope fusion antibodies (viral antigen-derived T-cell epitope peptide-fused cytotransmabs) inCT99-HPV_{E}†1-19 and inCT99-CMV_{P}†480-503 in which a peptide (CMV_{P}480-503) containing a CMV pp65-derived T-cell epitope is fused to a cytosol-penetrating antibody (cytotransmab);
FIG. 2 shows results of constructing and confirming a binding antigen for use in a library having improved affinity to the CMV_{P}495-503/HLA-A*02:01 complex,
FIG. 2a schematically showing a CMV/HLA-A*02:01 single-chain trimer (SCT) protein, in which the CMV_{P}495-503 peptide-β2m-HLA-A*02:01 complex was provided in the form of a single-chain trimer (SCT) via a peptide linker,
FIG. 2b showing the results of analysis of protein size and combination form through Coomassie Blue staining after SCT proteins were expressed and purified in animal cells (HEK293F) and then biotinylated and 3 µg of protein was separated through 12% SDS-PAGE under reducing or non-reducing conditions,
FIG. 2c showing the results of analysis of protein size and combination form through Coomassie Blue staining after purified peptide/HLA-A*02:01 SCT proteins, all of which were biotinylated to Avi-tag, were separated through SDS-PAGE under non-reducing conditions, performed using gel shift assay,
FIG. 2d showing the results of ELISA to investigate the ability of the purified CMV/HLA-A*02:01 SCT protein to bind to an antibody (BB7.2) recognizing the structure of HLA-A2 and the ability thereof to bind to the H9 antibody, which is a conventional TCR-like antibody;
FIG. 3 schematically shows a yeast cell surface H9 scFab CDR3 library construction strategy for improving affinity to the CMV_{P}495-503/HLA-A*02:01 complex,
FIG. 3a showing the construction of a library by introducing degenerate codons to the CDR3 portion of the heavy-chain variable region (VH) and the CDR3 portion of the light-chain variable region (VL) based on the sequence of the H9 clone known to bind to the CMV_{P}495-503/HLA-A*02:01 complex,
FIG. 3b schematically showing the constructed H9 scFab CDR3 library, which was fused to Agalp-Aga2p on the yeast cell surface and expressed in the form of single-chain Fab (antigen-binding fragment);

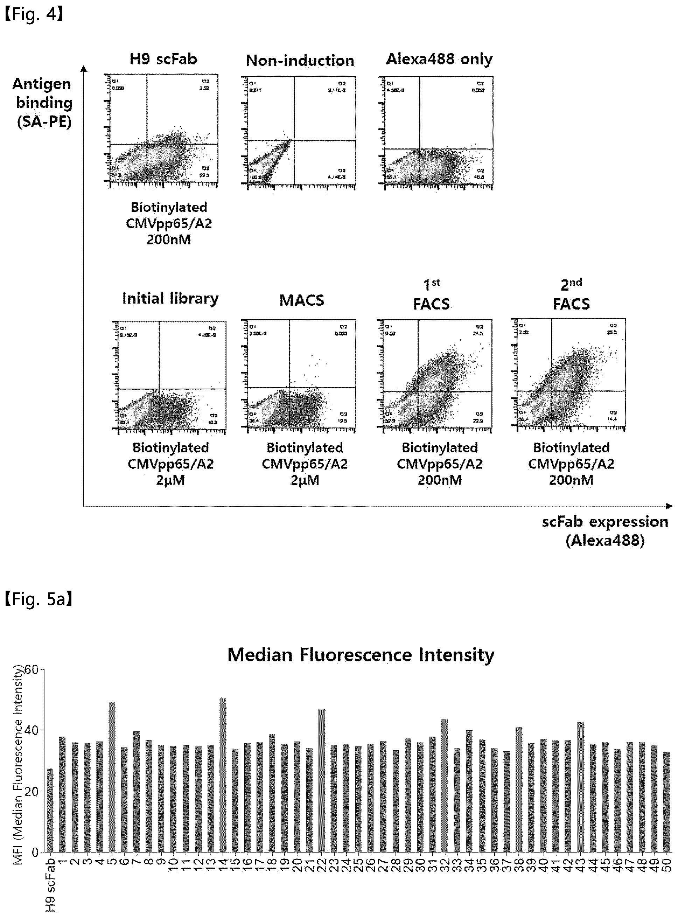

FIG. 4 shows the results of FACS analysis of pools bound to the CMV_{P}495-503/HLA-A*02:01 complex by performing MACS and FACS using the biotinylated CMV/HLA-A*02:01 SCT protein as a binding antigen in the constructed library, in which the expression level of single-chain Fab (scFab) and the binding to the biotinylated CMV/HLA-A*02:01 SCT protein may be analyzed, the expression level of single-chain Fab (scFab) on the yeast cell surface is compared, and the number of clones that bind to the biotinylated CMV/HLA-A*02:01 SCT protein and exhibit good expression of single-chain Fab (scFab) increases as MACS and FACS are repeated;
FIG. 5 shows the results of FACS identification of binding of the selected monoclones to the biotinylated CMV/HLA-A*02:01 SCT protein,
FIG. 5a showing the results of comparative analysis of a total of 50 monoclones with H9 scFab, in which the ability of each clone to bind to 2 nM of the biotinylated CMV/HLA-A*02:01 SCT protein was identified by the median fluorescence intensity shown in FACS, among which clones showing the top median fluorescence intensity were selected,
FIG. 5b showing the results of FACS analysis of the binding of the clones selected in FIG. 5a to 2 nM of the biotinylated CMV/HLA-A*02:01 SCT protein and the expression level of scFab on the yeast cell surface;
FIG. 6 shows the results of analysis of the converged clones #1 and #38, the heavy-chain variable region (VH) and light-chain variable region (VL) sequences of which were introduced into the heavy-chain constant region (CH1-hinge-CH2-CH3 domain) of mouse immunoglobulin 2a (mIgG2a) and the mouse kappa light-chain constant region (kappa domain) and expressed and purified in animal cells (HEK293F), in which 3 µg of protein was separated through SDS-PAGE under reducing or non-reducing conditions, and protein size and combination form were analyzed through Coomassie Blue staining;
FIG. 7 shows the results of ELISA measurement to determine the ability of #1 and #38 clones in IgG form expressed and purified in animal cells to bind to the CMV/HLA-A*02:01 SCT protein;
FIG. 8 shows the results of flow cytometry analysis to determine the ability of the H9 antibody and the H9 #1 and H9 #38 antibodies having improved affinity to bind to the CMV_{P}495-503/HLA-A*02:01 complex, after pulsing of 50 µM CMV_{P}495-503 peptide in cells having various cell-surface MHC-I (HLA-A*02:01) expression levels;
FIG. 9 shows the results of flow cytometry detection of the cell-surface CMV_{P}495-503/HLA-A*02:01 complex using the H9 antibody and the H9 #1 and H9 #38 antibodies having improved affinity, after inducing the formation of the cell-surface CMV_{P}495-503/HLA-A*02:01 complex by treating MDA-MB-231 cells with viral epitope fusion antibodies (viral antigen-derived T-cell epitope peptide-fused cytotransmabs) inCT99-HPV_{E}†1-19 and inCT99-CMV_{P}†480-503 in which a peptide (CMV_{P}480-503) containing a CMV pp65-derived T-cell epitope is fused to a cytosol-penetrating antibody (cytotransmab);

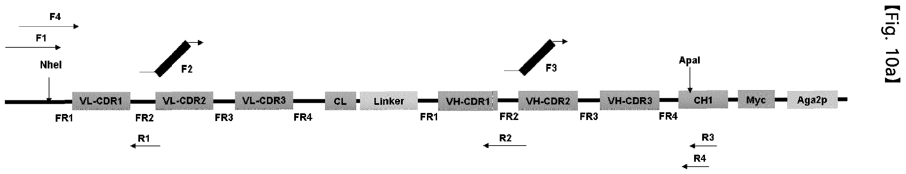

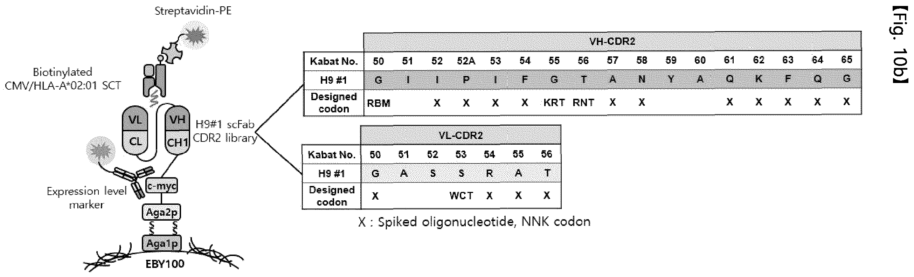

FIG. 10 schematically shows a yeast cell surface H9 #1 scFab CDR2 library construction strategy for improving affinity to the CMV_{P}495-503/HLA-A*02:01 complex,
FIG. 10a showing the construction of a library by introducing degenerate codons to the CDR2 portion of the heavy-chain variable region and the CDR2 portion of the light-chain variable region based on the sequence of the H9 #1 clone derived from the H9 scFab CDR3 library,
FIG. 10b schematically showing the constructed H9 #1 scFab CDR2 library, which was fused to Agalp-Aga2p on the yeast cell surface and expressed in the form of a single-chain Fab (scFab);
FIG. 11 shows the results of screening after FACS using the biotinylated CMV/HLA-A*02:01 SCT protein as a binding antigen in the constructed library;
FIG. 12 shows the results of FACS identification of binding of the selected monoclones to the biotinylated CMV/HLA-A*02:01 SCT protein,
FIG. 12a showing the results of comparative analysis of a total of 50 monoclones with H9 #1 scFab, in which the ability of each clone to bind to 0.5 nM of the biotinylated CMV/HLA-A*02:01 SCT protein was identified by the mean fluorescence intensity shown in FACS, among which clones showing the top median fluorescence intensity were selected,
FIG. 12b showing the results of FACS analysis of the binding of the clones selected in FIG. 12a to 0.5 nM of the biotinylated CMV/HLA-A*02:01 SCT protein and the expression level of scFab on the yeast cell surface;
FIG. 13 shows the results of analysis of the converged clones H9 #1-17 and H9 #1-30, the heavy-chain variable region (VH) and light-chain variable region (VL) sequences of which were introduced into the heavy-chain constant region (CH1-hinge-CH2-CH3 domain) of mouse immunoglobulin 2a (mIgG2a) and the mouse kappa light-chain constant region (kappa domain) and expressed and purified in animal cells (HEK293F), in which 3 µg of the protein was separated through SDS-PAGE under reducing or non-reducing conditions, and protein size and combination form were analyzed through Coomassie Blue staining;
FIG. 14 shows the results of ELISA measurement to determine the ability of H9 #1, H9 #1-17, and H9 #1-30 clones in IgG form expressed and purified in animal cells to bind to the CMV/HLA-A*02:01 SCT protein;
FIG. 15 shows the results of flow cytometry analysis to determine the ability of the H9 #1 antibody and the H9 #1-17 and H9 #1-30 antibodies having improved affinity to bind to the cell-surface CMV_{P}495-503/HLA-A*02:01 complex, after pulsing of 4 µM CMV_{P}495-503 peptide in an MDA-MB-231 cell line expressing cell-surface MHC-I (HLA-A*02:01);
FIG. 16 shows the results of flow cytometry detection of the cell-surface CMV_{P}495-503/HLA-A*02:01 complex using the H9 #1 antibody and the H9 #1-17 and H9 #1-30 antibodies having improved affinity, after inducing the formation of the cell-surface CMV_{P}495-503/HLA-A*02:01 complex by treating MDA-MB-231 cells with inCT99-CMV_{P}†480-503 and inCT99-HPV_{E}†1-19 fusion antibodies; and
FIG. 17 shows the results of determination of the affinity of TCR-like antibodies according to the present invention using Octet.

### [Mode for Invention]

Unless otherwise defined, all technical and scientific terms used herein have the same meanings as those typically understood by those skilled in the art to which the present invention belongs. In general, the nomenclature used herein is well known in the art and is typical.

The present invention pertains to a TCR (T-cell receptor)-like antibody specifically binding to a complex of MHC-I presenting a viral protein antigen-derived peptide or an antigen-binding fragment thereof.

The viral protein antigen-derived peptide preferably has the sequence shown in Table 1 below.

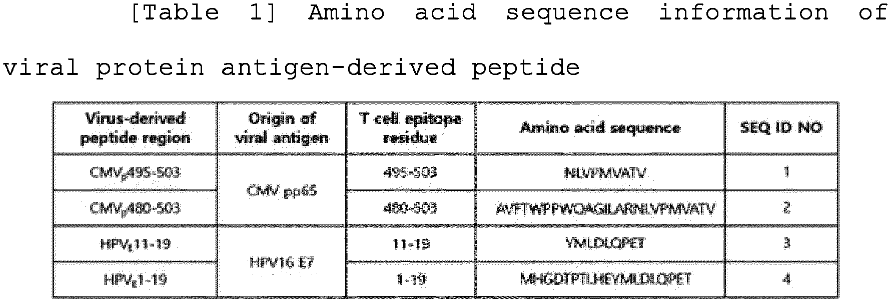

The present invention aims to provide a TCR-like antibody, which has improved affinity and is specific to a CMV_{P}495-503/HLA-A*02:01 complex in which the amino acid sequence 495-503-derived peptide (CMV_{P}495-503, 495-NLVPMVATV-503) of the pp65 protein of human cytomegalovirus (CMV) is presented to MHC-I, that is, HLA-A*02:01.

In the present invention, an intact antibody includes two heavy chains and two light chains.

As used herein, the term "heavy chain" is understood to include a full-length heavy chain and fragments thereof, the full-length heavy chain including a variable region domain VH including an amino acid sequence having a variable region sequence sufficient to confer specificity to an antigen and three constant region domains CH1, CH2, and CH3. Also, the term "light chain" is understood to include a full-length light chain and fragments thereof, the full-length light chain including a variable region domain VL including an amino acid sequence having a variable region sequence sufficient to confer specificity to an antigen and a constant region domain CL.

The constant region of an antibody is divided into a heavy-chain constant region and a light-chain constant region, and the heavy-chain constant region has gamma (γ), mu (µ), alpha (α), delta (δ), and epsilon (ε) types, and also has gamma 1 (γ1), gamma 2 (γ2), gamma 3 (γ3), gamma 4 (γ4), alpha 1 (α1), and alpha 2 (α2) subclasses. The constant region of the light chain has kappa (κ) and lambda (λ) types.

The variable region of an antibody is responsible for antigen binding. The variable regions in the light and heavy chains generally include complementarity-determining regions (CDRs) known to be important in antigen binding. Each variable region typically contains three highly variable loops (light-chain (LC) CDRs, including VL-CDR1, VL-CDR2, and VL-CDR3, and heavy-chain (HC) CDRs, including VH-CDR1, VH-CDR2, and VH-CDR3)). CDR boundaries for the antibody and the antigen-binding fragment disclosed herein may be defined or identified using a Kabat numbering scheme.

In the present invention, the antigen-binding fragment of an antibody is a fragment having an antigen-binding function, and includes Fab, F(ab'), F(ab')2, and Fv. Among antibody fragments, Fab (antigen-binding fragment) has a structure having light-chain and heavy-chain variable regions, a light-chain constant region, and a first heavy-chain constant region (CH1), and has one antigen-binding site. A single-chain antigen-binding fragment (scFab) is a polypeptide composed of light-chain and heavy-chain variable regions, a light-chain constant region, a first heavy-chain constant region (CH1), and a linker. In some cases, it may be stabilized through insertion of a cysteine residue. Fab' differs from Fab in that in that Fab' has a hinge region including at least one cysteine residue at the C-terminus of the heavy-chain CH1 domain. The F(ab')2 antibody is created by a disulfide bond between cysteine residues in the hinge region of Fab'.

Fv (variable fragment) is a minimal antibody fragment having only a heavy-chain variable region and a light-chain variable region. A two-chain Fv (two-chain variable fragment) is a fragment in which a heavy-chain variable region and a light-chain variable region are linked by a non-covalent bond, and a single-chain Fv (scFv) is a fragment in which a heavy-chain variable region and a light-chain variable region are generally linked by a covalent bond via a peptide linker therebetween, or are directly linked at the C-terminus, forming a dimeric structure, like the two-chain Fv. Such antibody fragments may be obtained using proteases (for example, Fab may be obtained by restriction-cleaving a whole antibody with papain, and the F(ab')2 fragment may be obtained by restriction-cleaving a whole antibody with pepsin), or may be prepared through genetic recombination technology.

In the present invention, the fragment of an antibody means each domain of the heavy chain or light chain of an antibody or a fragment thereof, for example, a heavy-chain variable region fragment (VH), heavy-chain constant region fragment (Fc), heavy-chain constant region domain (CH1, CH2, or CH3) fragment, antigen-binding fragment (Fab), single-chain variable fragment (scFv), light-chain constant region fragment (CL) or light-chain variable region fragment (VL), or fragments thereof.

In addition, the fragment of the antibody may be a monomer, a dimer, or a multimer.

Examples of the antibody may include, but are not limited to, monoclonal antibodies, non-specific antibodies, non-human antibodies, human antibodies, humanized antibodies, chimeric antibodies, single-chain Fvs (scFV), single-chain antibodies, Fab fragments, F(ab') fragments, disulfide-linked Fvs (sdFV), anti-idiotype (anti-Id) antibodies, and epitope-binding fragments of such antibodies.

A monoclonal antibody is an antibody obtained from a population of substantially homogeneous antibodies, in which the individual antibodies that make up the population are identical, except for possible naturally-occurring mutations that may be present at low frequency. A monoclonal antibody is highly specific and is induced against a single antigenic site. In contrast to typical (polyclonal) antibodies, which typically include different antibodies directed against different determinants (epitopes), each monoclonal antibody is directed against a single determinant on the antigen.

The term "epitope" refers to a protein determinant to which an antibody may specifically bind. The epitope is usually composed of a group of chemically active surface molecules, for example amino acids or sugar side chains, and generally has specific three-dimensional structural features and specific charge properties. Conformational and nonconformational epitopes are distinguished in that binding to the former, but not the latter, is lost in the presence of a denaturing solvent.

The monoclonal antibody may be IgG, IgM, IgA, IgD, or IgE, and for example, may be of an IgG1, IgG2, IgG3, IgG4, IgM, IgE, IgA1, IgA5, or IgD type. In addition, the light-chain constant region of the antibody may be a λ or κ type.

In the present invention, the TCR-like antibody includes a heavy-chain CDR1 of SEQ ID NO: 12, a heavy-chain CDR2 of SEQ ID NO: 13, a heavy-chain CDR3 selected from the group consisting of SEQ ID NOs: 15 and 16, a light-chain CDR1 of SEQ ID NO: 5, a light-chain CDR2 of SEQ ID NO: 6, and a light-chain CDR3 selected from the group consisting of SEQ ID NOs: 8 and 9, or

a heavy-chain CDR1 of SEQ ID NO: 12, a heavy-chain CDR2 selected from the group consisting of SEQ ID NOs: 17 and 18, a heavy-chain CDR3 of SEQ ID NO: 16, a light-chain CDR1 of SEQ ID NO: 5, a light-chain CDR2 selected from the group consisting of SEQ ID NOs: 10 and 11, and a light-chain CDR3 of SEQ ID NO: 9.

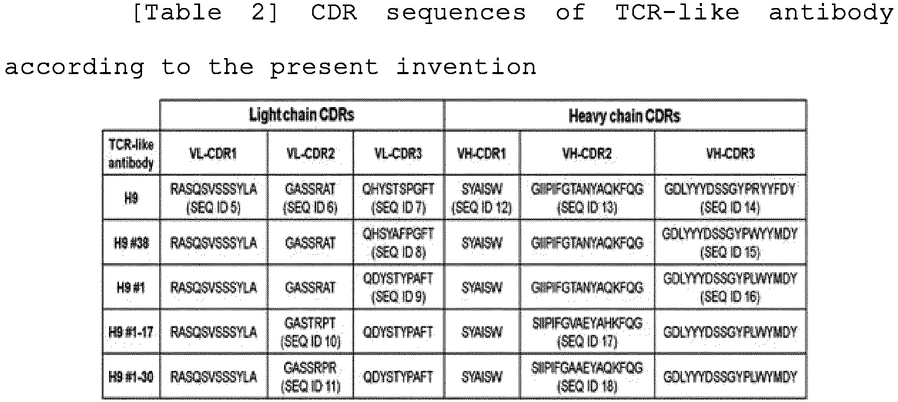

A "framework region" (FR) is a variable domain residue other than the CDR residue. Each variable domain typically has four FRs, identified as FR1, FR2, FR3, and FR4.

In the present invention, the TCR-like antibody may include a light-chain variable region selected from the group consisting of SEQ ID NOs: 20 to 23. It may include a heavy-chain variable region selected from the group consisting of SEQ ID NOs: 25 to 28.

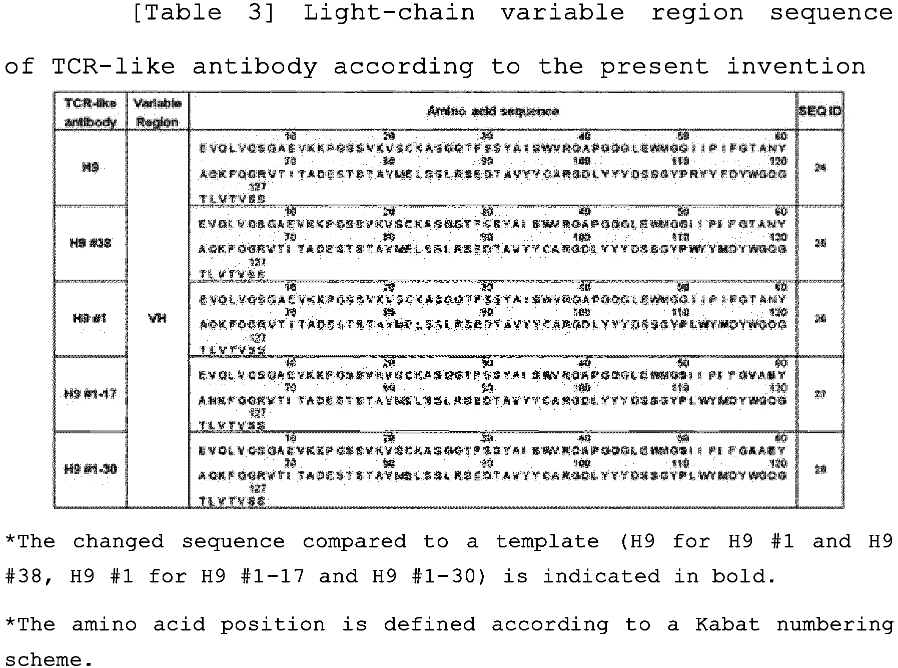

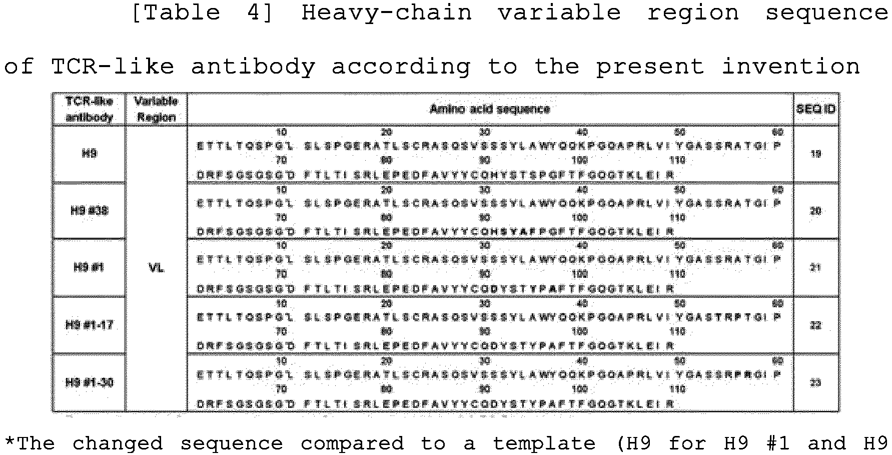

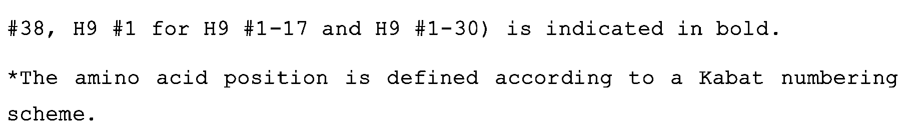

As used herein, the term "specifically binding" or "specific to" refers to a measurable and reproducible interaction that allows determination of the presence of a target in a population of heterogeneous molecules, including biological molecules, such as binding between a target and an antibody or antibody fragment. For example, an antibody or antibody fragment specifically binding to a target (which may be an epitope) is an antibody or antibody fragment that binds to the target with greater affinity, higher binding ability, more easily, and/or for a longer duration than to other targets.

The sequence according to the present invention may include not only the sequences described herein, but also biological equivalents thereof. For example, additional modifications may be made to the amino acid sequence of an antibody in order to further improve the binding affinity and/or other biological properties of the antibody. Such modifications include, for example, deletion, insertion, and/or substitution of the amino acid sequence residues of the antibody. The amino acid variations are based on the relative similarity of amino acid side-chain substituents, for example, hydrophobicity, hydrophilicity, charge, size, and the like. Based on analysis of the size, shape, and type of amino acid side-chain substituents, all of arginine, lysine, and histidine are positively charged residues, alanine, glycine, and serine have similar sizes, and phenylalanine, tryptophan, and tyrosine have similar shapes. Therefore, based on these considerations, arginine, lysine, and histidine may be regarded as biologically functional equivalents, alanine, glycine, and serine may be regarded as biologically functional equivalents, and phenylalanine, tryptophan, and tyrosine may be regarded as biologically functional equivalents.

Taking into consideration the above-described variations having equivalent biological activity, the antibody of the present invention or the nucleic acid molecule encoding the same is to be understood as including a sequence showing substantial identity to the provided sequence indicated by the sequence number. When the sequence of the present invention and any other sequence are aligned so as to correspond to each other as closely as possible and the aligned sequence is analyzed using an algorithm commonly used in the art, "substantial identity" refers to a sequence exhibiting at least 90% homology, preferably at least 95% homology, at least 96% homology, at least 97% homology, at least 98% homology, or at least 99% homology. Alignment methods for sequence comparison are known in the art. The NCBI Basic Local Alignment Search Tool (BLAST) is accessible through NBCI, etc., and may be used in conjunction with sequencing programs such as blastp, blastm, blastx, tblastn, and tblastx over the Internet. BLAST is available at www.ncbi.nlm.nih.gov/BLAST/. A method for comparing sequence homology using this program may be found at www.ncbi.nlm.nih.gov/BLAST/blast_help.html.

Based thereon, the antibody of the present invention or the antigen-binding fragment thereof may have 90%, 91%, 92%, 93%, 94%, 95%, 96%, 97%, 98%, 99%, or higher homology with a specified sequence or all of the sequences described in the specification. Such homology may be determined through sequence comparison and/or alignment using methods known in the art. For example, the percentage sequence homology of a nucleic acid or protein of the present invention may be determined using a sequence comparison algorithm (i.e. BLAST or BLAST 2.0), manual alignment, or visual inspection.

Another aspect of the present invention pertains to a nucleic acid encoding the antibody or the antigen-binding fragment thereof.

An antibody or an antigen-binding fragment thereof may be recombinantly produced by isolating a nucleic acid encoding the antibody of the present invention or the antigen-binding fragment thereof. The nucleic acid is isolated and inserted into a replicable vector for further cloning (DNA amplification) or further expression. In still another aspect, based thereon, the present invention pertains to a vector including the nucleic acid.

As used herein, the term "nucleic acid" has a meaning comprehensively encompassing DNA (gDNA and cDNA) and RNA molecules, and nucleotides, which are the basic building blocks of nucleic acids, include natural nucleotides as well as analogues in which sugar or base regions are modified. The sequence of the nucleic acid encoding the heavy-chain and light-chain variable regions of the present invention may be modified. Such modification includes addition, deletion, or non-conservative or conservative substitution of nucleotides.

DNA encoding the antibody is easily isolated or synthesized using typical procedures (e.g. using a polynucleotide probe capable of specifically binding to DNA encoding the heavy and light chains of the antibody). Many vectors are commercially available. A vector component generally includes, but is not limited to, at least one selected from among a signal sequence, an origin of replication, at least one marker gene, an enhancer element, a promoter, and a transcription termination sequence.

As used herein, the term "vector" refers to means for expressing a target gene in a host cell, including a plasmid vector, a cosmid vector, a viral vector such as a bacteriophage vector, adenoviral vector, retroviral vector or adeno-associated viral vector, and the like. In the vector, the nucleic acid encoding the antibody is operably linked with a promoter.

As used herein, the term "operably linked" means a functional linkage between a nucleic acid expression control sequence (e.g. a promoter, a signal sequence, or an array of transcriptional regulator binding sites) and a different nucleic acid sequence, whereby the control sequence serves to control the transcription and/or translation of the different nucleic acid sequence.

When a prokaryotic cell is used as a host, a strong promoter capable of promoting transcription (e.g. a tac promoter, lac promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiation of translation, and a transcription/translation termination sequence are generally included. In addition, for example, when a eukaryotic cell is used as a host, a promoter derived from the genome of a mammalian cell (e.g. a metallothionine promoter, β-actin promoter, human hemoglobin promoter or human muscle creatine promoter) or a promoter derived from a mammalian virus (e.g. an adenovirus late promoter, vaccinia virus 7.5K promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), or promoter of Rous sarcoma virus (RSV)) may be used, and a polyadenylation sequence is generally used as a transcription termination sequence.

In some cases, the vector may be fused with another sequence in order to facilitate purification of the antibody expressed therefrom. Examples of the sequence that is fused therewith include glutathione S-transferase (Pharmacia, USA), maltose-binding protein (NEB, USA), FLAG (IBI, USA), and 6x His (hexa-histidine; Qiagen, USA)).

The vector contains, as a selective marker, an antibiotic resistance gene that is commonly used in the art, for example, a gene conferring resistance to ampicillin, gentamicin, carbenicillin, chloramphenicol, streptomycin, kanamycin, geneticin, neomycin, or tetracycline.

Yet another aspect of the present invention pertains to a cell transformed with the vector described above. Examples of the cell used to produce the antibody of the present invention may include, but are not limited to, prokaryotic cells, yeast cells, and higher eukaryotic cells.

Prokaryotic host cells, such as *Escherichia coli, Bacillus subtilis* and *Bacillus thuringiensis* as strains belonging to the genus *Bacillus, Streptomyces, Pseudomonas* (e.g. *Pseudomonas putida*)*, Proteus mirabilis,* and *Staphylococcus* (e.g. *Staphylococcus carnosus*), may be used.

Here, animal cells are of greatest interest, and examples of useful host cell lines may include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

Still yet another aspect of the present invention pertains to a method of producing the antibody or the antigen-binding fragment thereof including (a) culturing the cells described above and (b) recovering an antibody or an antigen-binding fragment thereof from the cultured cells.

The cells may be cultured in various media. Any commercially available medium may be used as a culture medium without limitation. All other essential supplements known to those skilled in the art may be contained in appropriate concentrations. Culture conditions, such as temperature, pH, etc., are those already used for the host cells selected for expression, as will be apparent to those skilled in the art.

For the recovery of the antibody or the antigen-binding fragment thereof, impurities may be removed through, for example, centrifugation or ultrafiltration, and the resultant product may be purified using, for example, affinity chromatography. Other additional purification techniques, such as anion or cation exchange chromatography, hydrophobic interaction chromatography, hydroxyapatite chromatography, and the like, may be used.

A further aspect of the present invention pertains to a composition including T cells expressing the antibody or the antigen-binding fragment thereof as a chimeric antigen receptor (CAR).

The CAR may include an extracellular domain including an antigen-binding domain, a transmembrane domain, and an intracellular signaling domain. The extracellular domain may be connected to the transmembrane domain via a linker. The extracellular domain may also include a signal peptide.

The CAR includes a single-chain fragment variable region (scFv) of an antibody specific to a tumor-associated antigen (TAA) that is coupled via hinge and transmembrane regions to the cytoplasmic domain of a T-cell signaling molecule. Most common lymphocyte activation moieties include T-cell co-stimulatory (e.g. CD28, CD137, OX40, ICOS, and CD27) domains in tandem having a T cell-triggering (e.g. CD3ζ) moiety. CAR-mediated adoptive immunotherapy allows CAR-transplanted cells to directly recognize TAAs on target tumor cells in a non-HLA-restricted manner.

The present invention includes cells genetically modified to express CAR. The cells may be immune cells, preferably T cells.

Still a further aspect of the present invention pertains to a composition for preventing or treating cancer or an infectious disease including the antibody or the antigen-binding fragment thereof.

The present invention may address, for example, a pharmaceutical composition for the prevention or treatment of cancer or an infectious disease including (a) a pharmaceutically effective amount of the antibody according to the present invention or the antigen-binding fragment thereof and (b) a pharmaceutically acceptable carrier. In addition, the present invention pertains to a method of preventing or treating cancer or an infectious disease including administering the antibody according to the present invention or the antigen-binding fragment thereof in an effective amount required for a patient.

In the present invention, the cancer is selected from the group consisting of melanoma, lung cancer, liver cancer, glioblastoma, ovarian cancer, colorectal cancer, head and neck cancer, bladder cancer, kidney cancer, gastric cancer, breast cancer, metastatic cancer, Hodgkin's lymphoma, prostate cancer, and pancreatic cancer, but is not limited thereto.

As used herein, the term "treatment" refers to inhibiting or alleviating cancer or an infectious disease or one or more symptoms arising therefrom by administering the composition, as well as treating cancer or an infectious disease or preventing the progression thereof, which reverses the symptoms of the disease. In the present invention, the term "prevention" refers to any action that suppresses or delays the onset of cancer or an infectious disease by administration of the composition.

As used herein, the term "pharmaceutically acceptable carrier" refers to a carrier or diluent that does not stimulate the organism and does not inhibit the biological activity or properties of the administered compound. Examples of pharmaceutically acceptable carriers for compositions formulated in liquid solutions include saline, sterile water, Ringer's solution, buffered saline, albumin injection solution, dextrose solution, maltodextrin solution, glycerol, ethanol, and mixtures of one or more of these components, which are sterile and biocompatible, and other typical additives such as antioxidants, buffers, and bacteriostats may be added as necessary. In addition, diluents, dispersants, surfactants, binders, and lubricants may be additionally added to form an injectable formulation, such as an aqueous solution, suspension, emulsion, etc., pills, capsules, granules, or tablets.

The composition for preventing or treating cancer or an infectious disease including the antibody and the pharmaceutically acceptable carrier according to the present invention is applicable to any formulation containing the same as an active ingredient, and may be prepared as an oral or parenteral formulation. The pharmaceutical formulation of the present invention is provided in a form suitable for oral, rectal, nasal, topical (buccal and sublingual), subcutaneous, vaginal, or parenteral (intramuscular, subcutaneous, and intravenous) administration, or administration by inhalation or insufflation.

Formulations for parenteral administration containing the composition of the present invention as an active ingredient include injectable forms for subcutaneous injection, intravenous injection, or intramuscular injection, suppositories, or sprays such as aerosols for inhalation through the respiratory system. In order to prepare an injectable formulation, the composition of the present invention may be mixed with a stabilizer or a buffer in water to prepare a solution or suspension, which may then be formulated for unit administration in ampoules or vials. For suppository injection, it may be formulated into a composition for rectal administration such as a suppository or an enema containing a typical suppository base such as cocoa butter or another glyceride. In the case of a formulation for a spray, such as an aerosol, a propellant or the like may be blended with an additive to disperse the water-dispersed concentrate or wet powder.

"Administration" means introducing the pharmaceutical composition of the present invention to a patient through any suitable method. The composition of the present invention may be administered through various routes, either oral or parenteral, so long as it is able to reach the target tissue. Specifically, the composition may be administered in a typical manner via oral, rectal, topical, intravenous, intraperitoneal, intramuscular, intraarterial, transdermal, intranasal, inhalation, intraocular, or intradermal routes.

The method for treatment according to the present invention includes administering the composition of the present invention for preventing or treating cancer or an infectious disease in a pharmaceutically effective amount. It will be obvious to those skilled in the art that the total daily amount may be appropriately determined by a doctor within the scope of sound medical judgment. It is preferred that a specific therapeutically effective amount for a particular patient be differently applied depending on various factors including the type and extent of response to be achieved, the specific composition including whether other agents are used as necessary, the patient's age, body weight, general health status, gender, and diet, the time of administration, the route of administration, the secretion rate of the composition, the duration of treatment, and drugs used together or concurrently with the specific composition, and similar factors well known in the pharmaceutical field. Therefore, it is preferable to determine the effective amount of the composition for preventing or treating cancer suitable for the purpose of the present invention in consideration of the foregoing.

Yet a further aspect of the present invention pertains to a composition for diagnosing cancer or an infectious disease including the antibody or the antigen-binding fragment thereof. The present invention also pertains to a method of diagnosing cancer by treating a sample with the antibody or the antigen-binding fragment thereof. Using the antibody or the antigen-binding fragment thereof, radioimmunoassay, radioimmunoprecipitation, immunoprecipitation, immunohistochemical staining, ELISA (enzyme-linked immunosorbent assay), capture-ELISA, inhibitory or competitive assay, sandwich assay, flow cytometry, immunofluorescence staining, and immunoaffinity purification may be performed, but the present invention is not limited thereto.

A better understanding of the present invention may be obtained through the following examples. These examples are merely set forth to illustrate the present invention, and are not to be construed as limiting the scope of the present invention, as will be apparent to those of ordinary skill in the art.

### Example 1: Verification of TCR-like antibody H9 specific to CMV_{P}495-503/HLA-A*02:01 complex

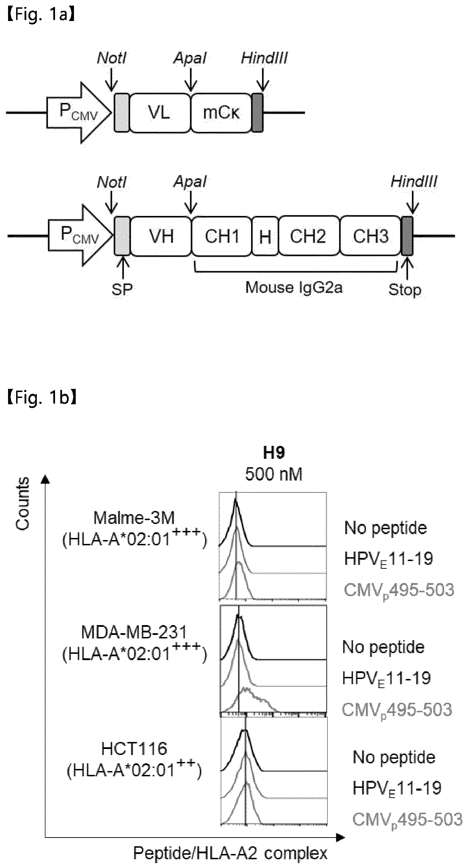

FIG. 1a schematically shows a vector for expressing, in animal cells (HEK293F), the heavy-chain variable region (VH) and the light-chain variable region (VL) of a TCR-like antibody H9 clone that specifically recognizes a CMV_{P}495-503/HLA-A*02:01 complex, which are introduced to the heavy-chain constant region (CH1-hinge-CH2-CH3 domain) of mouse immunoglobulin 2a (mIgG2a) and the mouse kappa light-chain constant region (kappa domain).

Specifically, the human heavy-chain variable region (VH) and human light-chain variable region (VL) sequences of the TCR-like antibody H9 clone specifically recognizing the CMV_{P}495-503/HLA-A*02:01 complex were prepared through gene synthesis (Bioneer, Korea). The VH gene was introduced into a heavy chain in which the heavy-chain constant region is mouse immunoglobulin G 2a through a PCR method to produce a DNA fragment, which was then cloned into an animal cell expression vector pcDNA3.4 with NotI and HindIII restriction enzymes. The VL gene was cloned into a light chain in which the light-chain constant region is the mouse kappa constant region in the same manner as above, thereby constructing a chimeric antibody.

FIG. 1b shows the results of flow cytometry analysis to determine the ability of the H9 antibody to bind to the CMV_{P}495-503/HLA-A*02:01 complex, after pulsing of 50 µM CMV_{P}495-503 peptide in cells having various cell-surface MHC-I (HLA-A*02:01) expression levels through expression and purification of the constructed chimeric H9 antibody.

Specifically, Malme-3M, MDA-MB-231, and HCT116 cells were prepared at a density of 3.0×10⁵ cells/ml in an RPMI medium containing 10% FBS and 1% ABAM antibiotic, and an amino acid sequence 11-19-derived peptide (HPV_{E}11-19, 11-YMLDLQPET-19) of HPV16 E7 protein, which is another virus-derived synthetic peptide capable of forming a complex with HLA-A*02:01 by including a CMV_{P}495-503 peptide, was added at a concentration of 50 µM thereto, followed by reaction at 5% CO₂ and 37°C for 3 hours. A negative control group not treated with the peptide was added with DMSO. After 3 hours, the supernatant containing peptide not bound to the cells was removed through centrifugation at 1300 rpm for 3 minutes, followed by washing with a FACS buffer (PBS containing 1% FBS) and then centrifugation at 1300 rpm for 3 minutes to remove the supernatant. For each sample, 1.5×10⁵ cells were prepared, and the H9 antibody obtained after expression and purification was diluted to a concentration of 500 nM in 100 µl of a FACS buffer and added thereto, followed by reaction at 4°C for 1 hour. After washing each sample with 1 ml of a FACS buffer, a F(ab')2 antibody (Jackson ImmunoResearch, USA) that specifically recognizes Alexa647 (red fluorescence)-conjugated mouse Fc (Jackson ImmunoResearch, USA) was diluted 1:600 in 100 µl of a FACS buffer, followed by reaction at 4°C for 30 minutes. Based on the results of flow cytometry analysis after washing with 1 ml of a FACS buffer, specific binding was observed only in MDA-MB-231 cells pulsed with the CMV_{P}495-503 peptide, but not to other peptide/MHC complexes. However, specific binding of the H9 antibody to the CMV_{P}495-503/HLA-A*02:01 complex was not observed in Malme-3M cells or in HCT116 cells in which the expression level of MHC-I (HLA-A*02:01) was low.

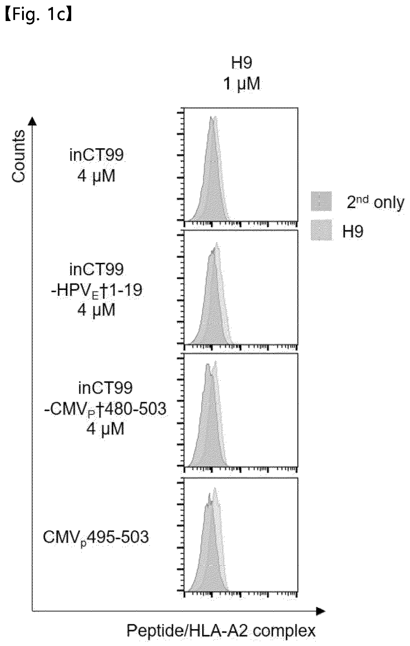

FIG. 1c shows the results of flow cytometry analysis to determine the ability of the H9 antibody to bind to the CMV_{P}495-503/HLA-A*02:01 complex, after inducing the formation of the cell-surface CMV_{P}495-503/HLA-A*02:01 complex by treating MDA-MB-231 cells with viral epitope fusion antibodies (viral antigen-derived T-cell epitope peptide-fused cytotransmabs) inCT99-HPV_{E}†1-19 and inCT99-CMV_{P}†480-503 in which a peptide (CMV_{P}480-503) containing a CMV pp65-derived T-cell epitope is fused to a cytosol-penetrating antibody (cytotransmab).

Information on the viral epitope fusion antibody (viral antigen-derived T-cell epitope peptide-fused cytotransmab) in which the peptide containing the viral antigen-derived T-cell epitope is fused to the cytosol-penetrating antibody used in this Example is shown in Table 5 below.

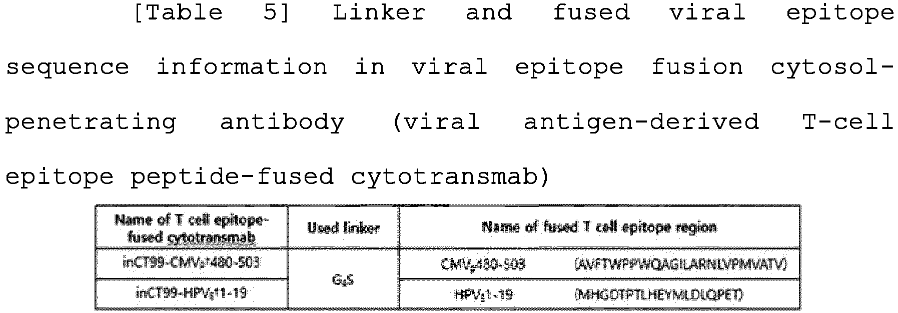

Specifically, in a 24-well plate, an MDA-MB-231 cell line was diluted at a density of 1.8×10⁵ cells/ml per well in an RPMI medium containing 10% FBS and 1% ABAM antibiotic, followed by culture at 37°C and 5% CO₂ for 12 hours, after which the medium was removed, and inCT99, inCT99-CMV_{P}†480-503, and inCT99-HPV_{E}†1-19 fusion antibodies were diluted to a concentration of 4 µM in 1 ml of a medium, followed by culture at 37°C and 5% CO₂ for 18 hours. After removing the medium, the cells were washed with DPBS and then treated with 100 µl of a trypsin-EDTA (Trypsin-EDTA) solution per well at 37°C for 2 minutes to separate the cells from the plate. 1 ml of an RPMI medium containing 10% FBS per well was added to neutralize trypsin-EDTA, and the cells were recovered and centrifuged at 1300 rpm for 3 minutes to remove the supernatant. The cells were washed with 1 ml of a FACS buffer and centrifuged to remove the supernatant. The cell pellets in each well were lysed with 200 µl of a FACS buffer and divided into two samples, one sample of which was used as a control containing only a secondary antibody. Each sample was added with the H9 antibody diluted to a concentration from 100 µl to 1 µM in a FACS buffer, followed by reaction at 4°C for 1 hour. After washing each sample with 1 ml of a FACS buffer, a F(ab')2 antibody that specifically recognizes Alexa647 (red fluorescence)-conjugated mouse Fc was diluted 1:600 in 100 µl of a FACS buffer, followed by reaction at 4°C for 30 minutes. Based on the results of flow cytometry analysis after washing with 1 ml of a FACS buffer, non-specific binding of the H9 antibody was observed in the experimental groups treated with inCT99 and inCT99-HPV_{E}†1-19, in addition to inCT99-CMV_{P}†480-503.

### Example 2: Preparation and verification of CMV_{P}495-503/HLA-A*02:01 complex protein

The conventional H9 clone, which is an antibody specifically binding to the CMV_{P}495-503/HLA-A*02:01 complex, was determined to show low affinity of about 300 nM through SPR (surface plasmon resonance). Accordingly, the CMV_{P}495-503/HLA-A*02:01 complex could not be detected in HCT116 cells having low cell-surface MHC-I (HLA-A*02:01) expression levels when treated with the H9 antibody. When cancer cells were treated with a viral epitope fusion antibody in which a peptide (CMV_{P}480-503) containing a CMV pp65-derived T-cell epitope is fused to a cytosol-penetrating antibody and then a CMV_{P}495-503/HLA-A*02:01 complex processed and presented in the cancer cells was detected, non-specific binding was also observed in other experimental groups at a detectable concentration, so a strategy for improving affinity by introducing a library to the CDR3 sequences of VH and VL of the H9 antibody was adopted. To this end, an antigen for use in screening antibodies having improved affinity by presenting a library on the surface of yeast cells was prepared.

In order to conventionally produce a TCR-like antibody, an antigen for immunizing mice is prepared in a manner in which alpha 1, 2, and 3 chain proteins and β2m protein constituting HLA-A*02:01 are expressed in E. *coli* to form an inclusion body, which is then added with a synthetic peptide, followed by refolding to create a trimer complex. However, since there is the case in which a protein was produced by linking peptide/p2m/HLA-A*02:01 to the N-terminus of the heavy-chain variable region of a monoclonal antibody via a peptide linker through an animal cell expression system (Truscott, Lybarger et al. 2007), a protein expression vector was constructed with reference thereto.

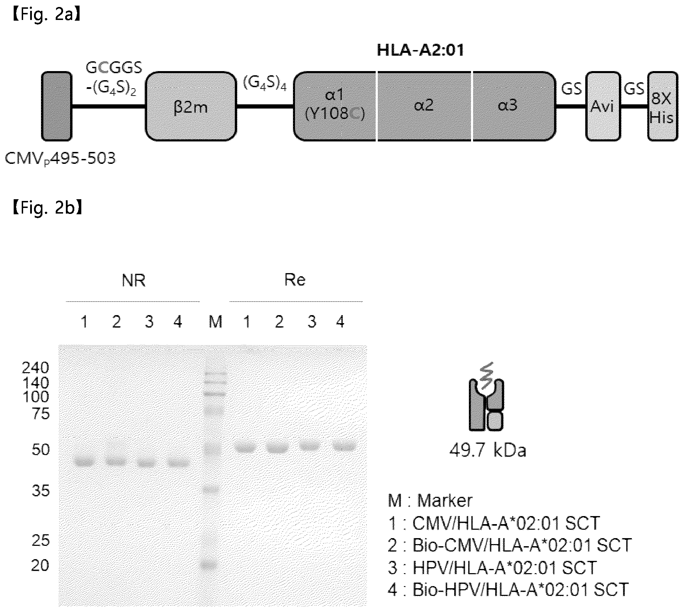

FIG. 2a schematically shows a protein in which a CMV_{P}495-503 peptide/p2m/HLA-A*02:01 is provided in the form of a single-chain trimer (SCT) via a peptide linker.

Specifically, DNA encoding a secretory signal peptide and a CMV_{P}495-503 peptide and β2m were linked at the 5' end via a GCGGS-(G₄S)₂ peptide linker, and β2m and HLA-A*02:01 extracellular domain α1 (Y108C)-α2-α3 (residues 25-298) were linked via a (G₄S)₄ peptide linker. At the C-terminus of HLA-A*02:01, DNA in a form in which an Avi-tag and an 8X His-tag were fused using a GS peptide linker was constructed through PCR and cloned into the animal cell expression vector pcDNA3.4 with NotI and HindIII restriction enzymes. A disulfide bond with Cys of the GCGGS-(G₄S)₂ peptide linker was formed by substituting Cys for Tyr at position 108 in the HLA-A*02:01 α1 domain in order to maintain stable binding of the single-chain CMV_{P}495-503 peptide to the HLA peptide-binding site. A protein in which the CMV_{P}495-503 peptide was replaced with the HPV_{E}11-19 peptide was constructed in the same manner as above.

FIG. 2b shows the results of analysis of protein size and combination form through Coomassie Blue staining after SCT proteins were expressed and purified in animal cells (HEK293F) and then biotinylated to thus separate 3 µg of protein through 12% SDS-PAGE under reducing or non-reducing conditions.

Specifically, upon transfection of 100 mL thereof into a shake flask, HEK293F cells were seeded at a density of 2.0×10⁶ cells/ml in 90 mL of a medium, followed by culture at 120 rpm, 8% CO₂, and 37°C. In order to produce a protein, a CMV_{P}495-503/β2m/HLA-A*02:01-Avi-8X His plasmid was diluted to 125 µg (1.25 µg/ml) in 5 ml of a FreeStyle 293 expression medium, filtered, mixed with 5 ml of a medium diluted with 375 µg (3.75 µg/ml) of PEI, and allowed to react at room temperature for 10 minutes. Thereafter, the reacted mixed medium was added to the cells seeded in 90 ml, followed by culture in an incubator at 120 rpm and 8% CO₂ for 6 days. The protein was purified using a Ni-NTA resin that specifically purifies the His-tag-fused protein from the collected cell culture supernatant with reference to a standard protocol. 100 ml of the cell culture supernatant was mixed at 1:1 with a buffer containing 50 mM Tris-HCl, 300 mM NaCl, and 10 mM imidazole at a pH of 8.0, added with 1 ml of a Ni-NTA resin, and then rotated at 4°C for 1 hour. The Ni-NTA resin was washed with 50 ml of a buffer containing 50 mM Tris-HCl, 300 mM NaCl, and 10 mM imidazole at a pH of 8.0, and then with 50 ml of a buffer containing 50 mM Tris-HCl, 300 mM NaCl, and 30 mM imidazole at a pH of 8.0. Protein was eluted using an elution buffer containing 50 mM Tris-HCl, 300 mM NaCl, and 200 mM imidazole at a pH of 8.0. The eluted fractions were collected, exchanged with a DPBS buffer through a PD-10 desalting column (GE healthcare), and concentrated using a 30K centrifugal filter tube (Corning). The purified protein was measured for absorbance at a wavelength of 562 nm using a solution in a BCA protein assay kit (Thermo), and then quantified according to the standard curve. Upon transfection of 100 ml thereof, a yield of about 9 mg was obtained.

In order to biotinylate the purified protein, a birA enzyme kit was used for the Avi-Tag portion of the antigen. Specifically, 1.8 mg of protein in a DPBS buffer was prepared and replaced with a reaction buffer containing 10 mM Tris-HCl at a pH of 8.0 using a PD-10 desalting column. In order to optimize the enzyme reaction, the antibody was concentrated to 40 µM or more using a centrifugal filter tube. The 10X reagent provided in the kit was mixed with the protein so that the final concentrations were 50 mM Bicine, 50 µM d-Biotin, 10 mM MgOAc, and 10 mM ATP and the antibody concentration was 40 µM. Here, 2.5 µg of BirA enzyme was used for 10 nmol of the protein substrate, and the reaction was carried out in a water bath at 30°C for 40 minutes. After the reaction, the protein buffer was replaced with DPBS using a PD-10 desalting column. The protein was quantified through BCA protein assay, and the yield after reaction using 1.8 mg of protein was about 1.7 mg.

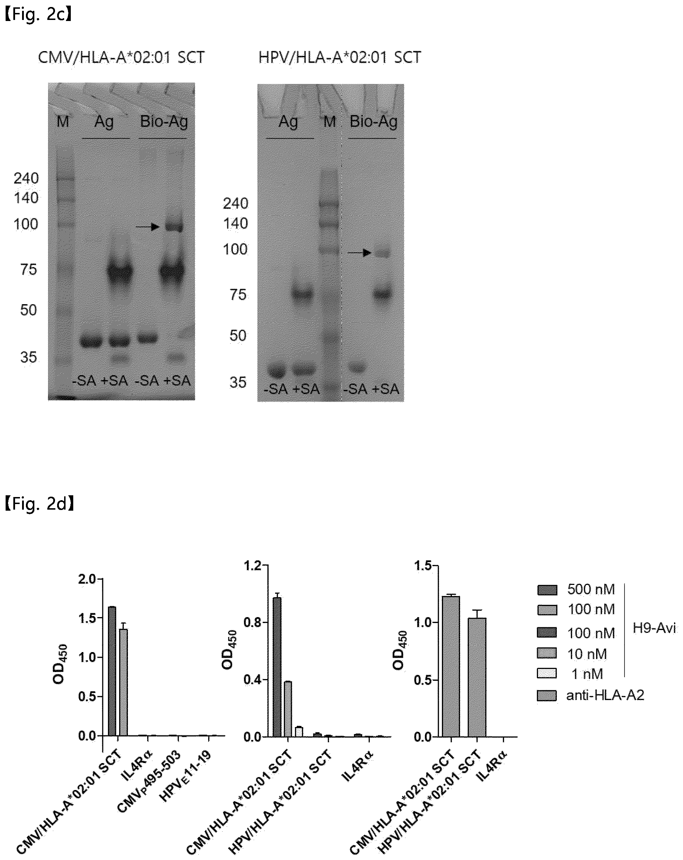

FIG. 2c shows the results of analysis of protein size and combination form through Coomassie Blue staining after the purified SCT proteins, all of which were biotinylated to Avi-tag, were separated through SDS-PAGE under non-reducing conditions, performed using gel shift assay.

Specifically, 3 µg of protein was prepared, diluted with SA in a number of moles corresponding to 4 times the number of moles thereof in a DPBS buffer, and allowed to react at room temperature for 30 minutes to form a complex. When the protein of about 49.7 kDa and the SA of about 52.8 kDa were formed into a complex, the size thereof was shifted and located at 100 kDa on SDS-PAGE. It was observed that all proteins were formed into a complex with SA and thus almost 100% of the reacted proteins were biotinylated.

FIG. 2d shows the results of ELISA to investigate the ability of the purified CMV/HLA-A*02:01 SCT protein to bind to the anti-HLA-A2 antibody and the ability thereof to specifically bind to the TCR-like antibody H9.

ELISA was performed to determine whether the CMV/HLA-A*02:01 SCT protein prepared through the above method had the ability to bind to the anti-HLA-A2 antibody or to specifically bind to the TCR-like antibody H9. Specifically, CMV/HLA-A*02:01 SCT protein, HPV/HLA-A*02:01 SCT protein, and IL (interleukin)-4 receptor alpha protein at concentrations of 10 µg/ml and CMV_{P}495-503 peptide and HPV_{E}11-19 peptide at concentrations of 1 µg/ml were allowed to bind at room temperature for 1 hour in a PBS buffer at a pH of 7.4 on EIA/RIA plates (COSTAR Corning), washed three times with 0.05% PBST (PBS at a pH of 7.4, 0.05% Tween20) for 10 minutes, and then allowed to bind at room temperature for 1 hour in a blocking buffer (PBS at a pH of 7.4, 2% bovine serum albumin (BSA)). Thereafter, the H9-Avi antibody diluted to each of 500 nM, 100 nM, 10 nM, and 1 nM in a blocking buffer and the anti-HLA-A2 antibody diluted to 2 µg/ml were allowed to bind at room temperature for 1 hour and washed three times with a 0.05% PBST buffer for 10 minutes. An HRP-conjugated anti-mouse Fc antibody as a labeling antibody was diluted 1:4000 in a blocking buffer and allowed to bind thereto. After reaction with a TMB ELISA solution, the reaction was completed with H₂SO₄, and absorbance was measured at a wavelength of 450 nm.

Through the above experiment, proper formation of the structure of the purified CMV/HLA-A*02:01 SCT protein was confirmed through binding of the anti-HLA-A2 antibody (BB7.2) that recognizes the structure of HLA-A2 and concentration-dependent binding of the H9 antibody.

### Example 3: Design and construction of yeast H9 single-chain Fab (scFab) CDR3 library to improve affinity to CMV/HLA-A*02:01

The T-cell receptor is composed of α-polypeptide and β-polypeptide chains. Each chain has genes, called a variable region (V region), a junction region (J region), and a constant region (C region), and the β-chain additionally has a diversity region (D region). In the variable regions (V regions) of the α- and β-chains, there are three hypervariable regions called complementarity-determining regions (CDRs). In general, the CDR3 region has the most diversity among these regions, and plays the most important role in specific recognition of an antigen (Rubtsova, Scott-Browne et al. 2009). Accordingly, in the present invention, by introducing the library to the CDR3 (VH-CDR3) of the heavy-chain variable region (VH) and the CDR3 (VL-CDR3) of the light-chain variable region (VL) of the H9 clone, an attempt was made to isolate a clone having higher affinity to the antigen than a conventional H9 clone.

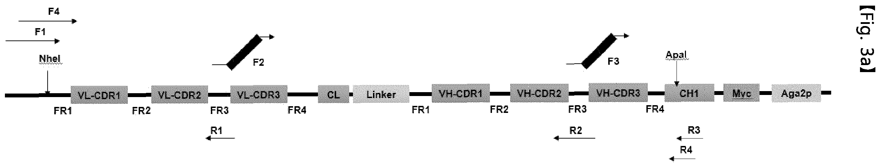

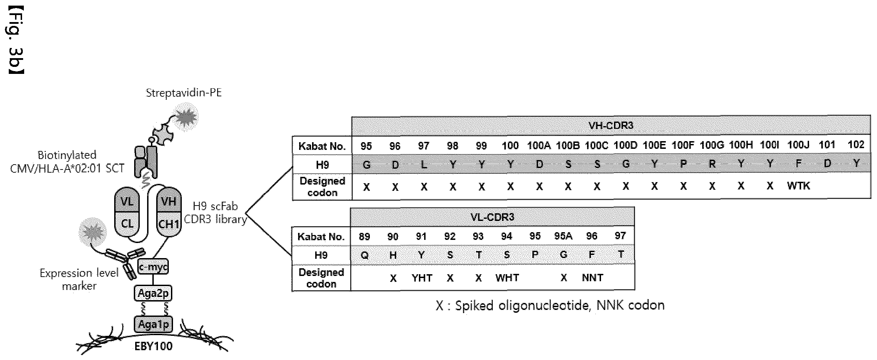

To this end, as shown in FIG. 3b, the antigen-binding fragment (Fab) of the H9 antibody was cloned in the form of a single-chain antigen-binding fragment (scFab) connected via a linker and then degenerate codons were introduced to a forward primer using the heavy-chain variable region (VH) (VH-CDR3) and CDR3 (VL-CDR3) of the light-chain variable region (VL) as a template, thereby constructing a library. In order to obtain as many clones as possible, the library was constructed with all NNK codons except for the portion corresponding to three residues at the C-terminus of the heavy-chain variable region CDR3. The sequence was conserved with aspartic acid and tyrosine in the two C-terminal residues of most antibody CDR3 regions, so this region was excluded. In addition, WTK, which is a degenerate codon composed of phenylalanine, isoleucine, methionine, and leucine, was selected so that phenylalanine and methionine, which frequently appear in most antibody sequences, could be located in the 3^{rd} residue at the C-terminus. Likewise, the light-chain variable region CDR3 was constructed with degenerate codons in consideration of amino acids that are conserved in most human antibodies.

In addition, a reverse primer was synthesized in the CH1 portion of the antibody heavy-chain constant region (Fc) fragment. Each of the forward and reverse primers includes a portion identical to the vector sequence of 50 base pairs or more so as to enable homologous recombination in yeast cells.

Library DNA was prepared by performing DNA polymerase chain reaction (PCR). Using, as a template, a pYDS-H9 scFab vector in which the light-chain variable region and heavy-chain variable region sequences of the H9 clone were inserted into a pYDS vector (Baek and Kim 2014) capable of expressing scFab on the yeast cell surface, DNA was amplified using the forward and reverse primers, and the amplified DNA (120 µg in total, 12 µg per transformation) was subjected to electroporation ten times on yeast together with the vector DNA (40 µg in total, 4 µg per transformation) obtained by treating the pYDS vector with NheI and ApaI restriction enzymes, thereby constructing a library. Subsequently, the library and the vector were allowed to bind in yeast cells through homologous recombination. The size of the library was determined to be 1.5×10⁷ by measuring the number of colonies grown in a selective medium depending on the selection factor present in the vector after serial dilution.

### Example 4: Selection of clones having improved affinity to CMV/HLA-A*02:01 SCT protein in constructed yeast H9 scFab VH-/VL-CDR3 library

FIG. 4 shows the process of selecting clones that specifically bind to the CMV/HLA-A*02:01 SCT protein constructed in Example 2 in the constructed yeast H9 CDR3 scFab library. Specifically, MACS (magnetic activated cell sorting) and FACS (fluorescence activated cell sorting) were performed respectively once and twice using the CMV/HLA-A*02:01 SCT protein prepared as in Example 2. 2 µM of the biotinylated CMV/HLA-A*02:01 SCT protein was allowed to bind to the H9 scFab VH-/VL-CDR3 library expressed on the yeast cell surface at 25°C for 1 hour. Thereafter, washing once with a MACS buffer and then reaction with streptavidin-microbead (Miltenyi Biotec Inc., Germany) at 4°C for 10 minutes were carried out to impart magnetism to the biotinylated CMV pp65/MHC complex bound to the yeast cell surface scFab library. Thereafter, primary MACS was performed and yeast cell surface scFab library clones capable of binding to the CMV/HLA-A*02:01 SCT protein were selected.

The selected clones were subjected to FACS, so the yeast cell surface expression level was confirmed through a mouse antibody 9e10 clone recognizing c-myc tag and an antibody recognizing Alexa488-conjugated mouse Fc, and biotinylated antigen binding was confirmed using PE-conjugated streptavidin (streptavidin-R-phycoerythrin conjugate (SA-PE), Thermo), thereby selecting clones having high scFab expression and high ability to bind to the biotinylated CMV/HLA-A*02:01 SCT protein in the state in which the concentration of the biotinylated CMV/HLA-A*02:01 SCT protein was decreased. This procedure was repeated twice.

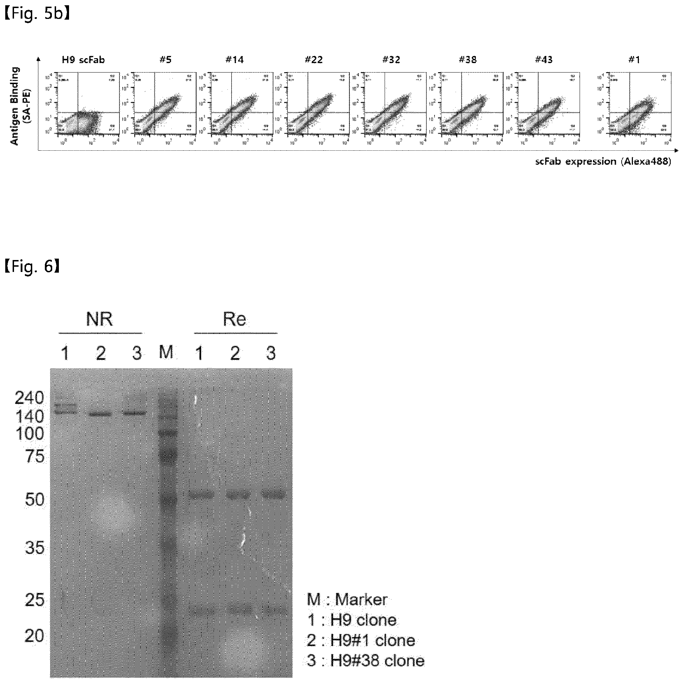

FIG. 5 shows the results of FACS analysis after binding of a total of 50 individual clones isolated from the library to 2 nM of the biotinylated CMV/HLA-A*02:01 SCT protein. When the #14, #22, #32, #38, and #43 clones having excellent scFab expression level and high antigen-binding strength and the #1 clone having a slightly different graph pattern from the remaining 49 clones were selected and sequenced, all clones except the #38 clone expressed scFab encoding the same amino acid sequence as #1.

Table 6 below shows the CDR amino acid sequences of the light-chain variable region and the heavy-chain variable region of the two converged clones. The changed amino acid sequence compared to the sequence of the H9 clone is indicated in bold.

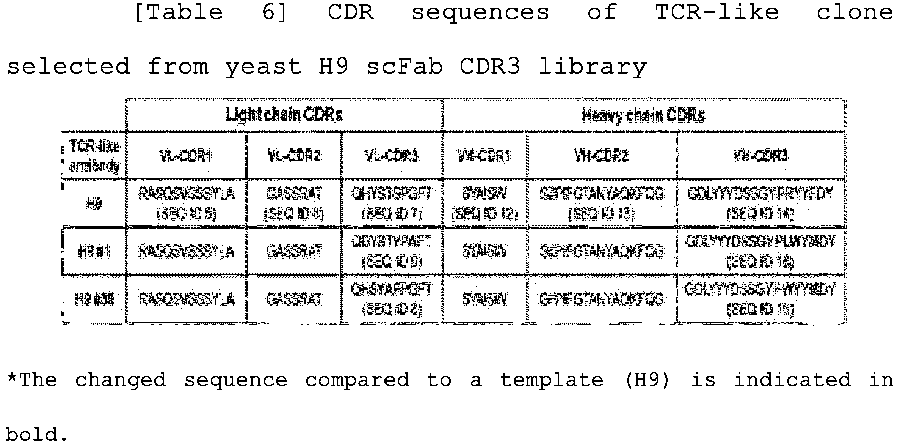

### Example 5: IgG conversion and identification of scFab clones having improved affinity to CMV/HLA-A*02:01 SCT protein

The heavy-chain variable region (VH) and light-chain variable region (VL) sequences of the selected H9 #1 and H9 #38 clones were introduced into the pcDNA3.4 vector encoding the heavy-chain constant region (CH1-hinge-CH2-CH3 domain) of mouse immunoglobulin 2a (mIgG2a) and the mouse kappa light-chain constant region (kappa domain).

FIG. 6 shows the results of analysis of protein size and combination form through Coomassie Blue staining after the H9 #1 and H9 #38 antibodies in which the affinity of the TCR-like antibody H9 was improved were expressed and purified in animal cells (HEK293F) and then 3 µg of protein was separated through 12% SDS-PAGE under reducing or non-reducing conditions. Respective yields of H9, H9 #1, and H9 #38 were 27.8 mg, 20.4 mg, and 8.51 mg per 1 of the HEK293F animal cell culture.

### Example 6: Evaluation of ability of selected TCR-like antibodies to bind to CMV/HLA-A*02:01 SCT protein

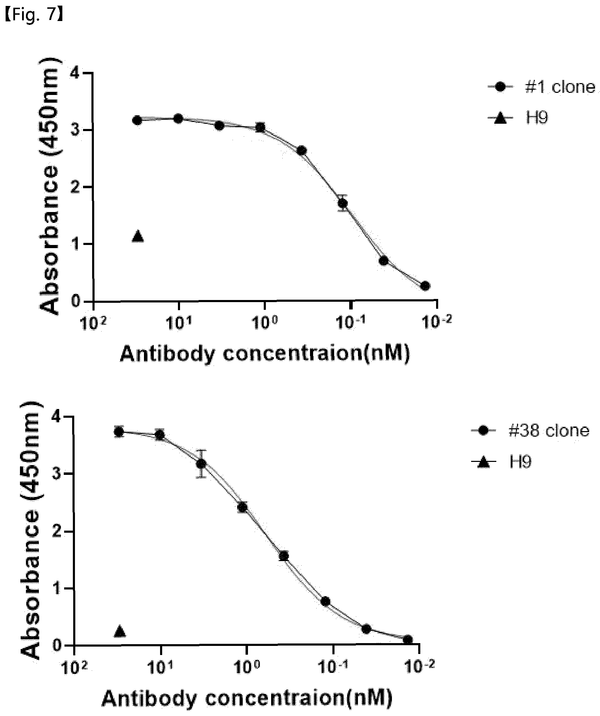

FIG. 7 shows the results of ELISA measurement to determine the ability of H9 #1 and H9 #38 in IgG form expressed and purified in animal cells to bind to CMV/HLA-A*02:01 SCT protein.

Specifically, EIA/RIA plates were coated with CMV/HLA-A*02:01 SCT protein at a concentration of 10 µg/ml in a PBS buffer at a pH of 7.4 at room temperature for 1 hour, and then washed three times with 0.05% PBST. Thereafter, binding was carried out in a blocking buffer at room temperature for 1 hour. The H9, H9 #1, and H9 #38 antibodies were diluted in a blocking buffer, allowed to bind at room temperature for 1 hour, and washed three times with a 0.05% PBST buffer. An HRP-conjugated anti-mouse Fc antibody as a labeling antibody was diluted 1:4000 in a blocking buffer and allowed to bind thereto. After reaction with a TMB ELISA solution, the reaction was completed with H₂SO₄, and absorbance was measured at a wavelength of 450 nm.

Based on the results of ELISA of the CMV/HLA-A*02:01 SCT protein in a concentration-dependent manner, compared to H9 in IgG form, the H9 #1 and H9 #38 clones in IgG form were confirmed to bind to the CMV/HLA-A*02:01 SCT protein with remarkably high affinity.

### Example 7: Evaluation of ability of selected TCR-like antibodies to bind to cell-surface CMV_{P}495-503/HLA-A*02:01 complex

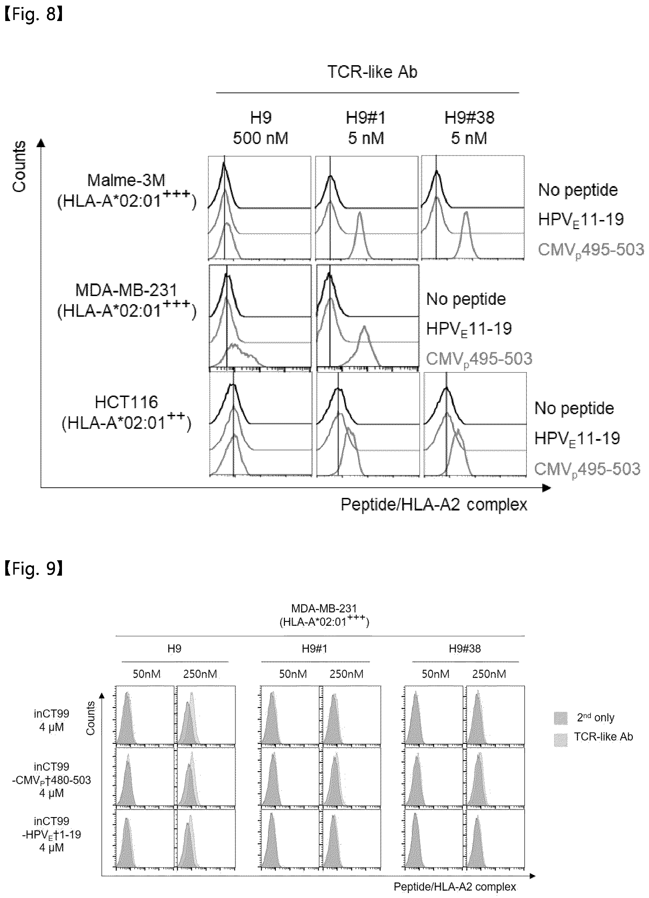

FIG. 8 shows the results of flow cytometry analysis to determine the ability of the H9 antibody and the H9 #1 and H9 #38 antibodies having improved affinity to bind to the CMV_{P}495-503/HLA-A*02:01 complex, after pulsing of 50 µM CMV_{P}495-503 peptide in cells having various cell-surface MHC-I (HLA-A*02:01) expression levels.

Specifically, peptide pulsing was performed in a Malme-3M melanoma cell line, an MDA-MB-231 breast cancer cell line, and a HCT116 colorectal cancer cell line in the same manner as in Example 1. After completion of pulsing, the cells were washed with a FACS buffer and centrifuged at 1300 rpm for 3 minutes to remove the supernatant. For each sample, 1.5×10⁵ cells were prepared, added with H9 diluted to 500 nM and H9#1 and H9#38 clones diluted to 5 nM in 100 µl of a FACS buffer, and allowed to react at 4°C for 1 hour. After washing each sample with 1 ml of a FACS buffer, a F(ab')2 antibody that specifically recognizes Alexa647 (red fluorescence)-conjugated mouse Fc was diluted 1:600 in 100 µl of a FACS buffer, followed by reaction at 4°C for 30 minutes. Based on the results of flow cytometry analysis after washing with 1 ml of a FACS buffer, it was confirmed that the experimental group treated with HPV_{E}11-19 peptide did not show binding of TCR-like antibodies in any cell lines, compared to the experimental group not treated with the peptide, and also that specific binding appeared only in the experimental group treated with the CMV_{P}495-503 peptide, indicating specificity of the TCR-like antibodies to the CMV_{P}495-503/HLA-A*02:01 complex. In addition, the H9 #1 and H9 #38 antibodies having improved affinity were capable of detecting a larger amount of the CMV_{P}495-503/HLA-A*02:01 complex even when reacted at a concentration 100 times lower than that of the H9 antibody, indicating that the affinity thereof was improved. In particular, the CMV_{P}495-503/HLA-A*02:01 complex on the surface of Malme-3M and HCT116 cells, which could not be detected by the H9 antibody in Example 1, was effectively detected, and the complex formation proportional to the cell surface expression level of HLA-A2 could be compared in three cell lines.

### Example 8: Detection of cell-surface CMV_{P}495-503/HLA-A*02:01 complex using selected TCR-like antibodies

When the MDA-MB-231 cell line was treated with the inCT99-CMV_{P}†480-503 fusion antibody, whether the formation of the cell-surface CMV_{P}495-503/HLA-A*02:01 complex could be induced was analyzed through flow cytometry using H9 #1 and H9 #38 antibodies in which the affinity of H9 was improved.

Specifically, in a 24-well plate, an MDA-MB-231 cell line was diluted at a density of 1.8×10⁵ cells/ml per well in an RPMI medium containing 10% FBS and 1% ABAM antibiotic and cultured at 37°C and 5% CO₂ for 12 hours, after which the medium was removed, and inCT99, inCT99-CMV_{P}†480-503, and inCT99-HPV_{E}†1-19 fusion antibodies were diluted to a concentration of 4 µM in 1 ml of a medium, followed by culture at 37°C and 5% CO₂ for 18 hours. Thereafter, the medium was removed, and the cells were washed with DPBS and then treated with 50 µl of a trypsin-EDTA solution per well at 37°C for 2 minutes and thus separated from the plate. 1 ml of an RPMI medium containing 10% FBS per well was added to neutralize trypsin-EDTA, and the cells were recovered and centrifuged at 1300 rpm for 3 minutes to remove the supernatant. The cells were washed with 1 ml of a FACS buffer and centrifuged to remove the supernatant. The cell pellets in each well were lysed with 200 µl of a FACS buffer and divided into two samples, one sample of which was used as a control containing only a secondary antibody. Each sample was added with H9, H9 #1, and H9 #38 at 50 nM and 250 nM in 100 µl of a FACS buffer and then allowed to react at 4°C for 1 hour. After washing each sample with 1 ml of a FACS buffer, a F(ab')2 antibody that specifically recognizes Alexa647 (red fluorescence)-conjugated mouse Fc was diluted 1:600 in 100 µl of a FACS buffer, followed by reaction at 4°C for 30 minutes. Based on the results of flow cytometry analysis after washing with 1 ml of a FACS buffer, the H9 antibody did not detect the CMV_{P}495-503 peptide/HLA-A*02:01 complex, but the H9 #1 and H9 #38 clones having improved affinity were able to detect the CMV_{P}495-503/HLA-A*02:01 complex upon reaction at a concentration of 250 nM. However, the detection intensity was very weak because the expression level of the cell-surface CMV_{P}495-503/HLA-A*02:01 complex was too low, so additional affinity improvement was determined to be required.

### Example 9: Design and construction of yeast H9 #1 single-chain Fab (scFab) VH-/VL-CDR2 library for improving affinity to CMV/HLA-A*02:01

In order to isolate clones having much higher affinity to the CMV_{P}495-503 peptide/HLA-A*02:01 complex than the H9 #1 clone having high ability to bind to the cell-surface CMV_{P}495-503/HLA-A*02:01 complex in Example 7, a library was constructed by introducing degenerate codons to the CDR2 sequences of the heavy-chain variable region and the light-chain variable region using H9 #1 as a template. As in Example 3, in order to obtain as many clones as possible, the library was constructed mainly with NNK degenerate codons, but degenerate codons were designed in consideration of amino acids that are conserved in most human antibodies with reference to the IMGT database. In the light-chain variable region CDR2, the 1^{st}, 2^{nd}, 7^{th}, and 8^{th} amino acids from the N-terminus were constructed with RBM, ATC, KRT, and RNT degenerate codons, respectively, and the 11^{th} and 12^{th} amino acids were conserved with tyrosine and alanine, respectively. Likewise, the light-chain variable region CDR2 was constructed mainly with NNK degenerate codons, the 2^{nd} and 3^{rd} amino acids from the N-terminus were conserved with alanine and serine, and the 4^{th} amino acid was constructed with a WCT degenerate codon capable of being encoded with serine or tyrosine. Other details with regard to the experimental method were the same as in Example 3.

The size of the library was determined to be 1.3×10⁷ by measuring the number of colonies grown in the selective medium depending on the selection factor present in the vector after serial dilution.

### Example 10: Selection of clones having improved affinity to CMV/HLA-A*02:01 SCT protein in constructed yeast H9 #1 scFab VH-/VL-CDR2 library

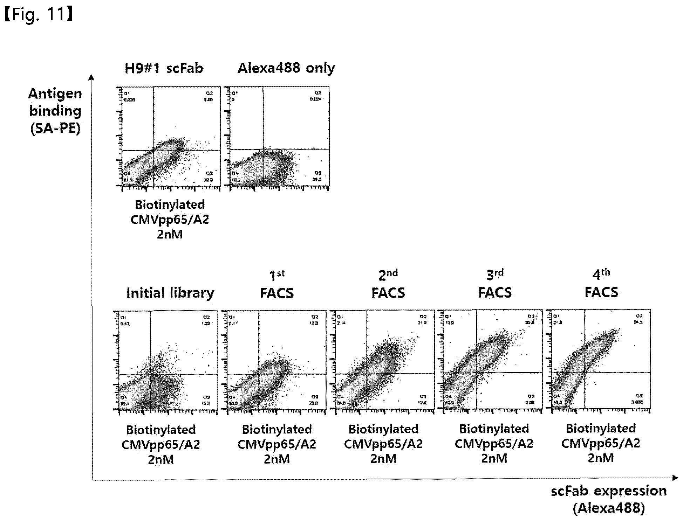

FIG. 11 shows a process of selecting clones that specifically bind to the CMV/HLA-A*02:01 SCT protein constructed in Example 9 in the constructed yeast H9 #1 CDR2 scFab library. Specifically, FACS (fluorescence activated cell sorting) was performed four times using the CMV/HLA-A*02:01 SCT protein prepared as in Example 9. 2 nM of the biotinylated CMV/HLA-A*02:01 SCT protein was allowed to bind to the H9 #1 CDR2 scFab library expressed on the yeast cell surface at 25°C for 1 hour. Thereafter, a yeast cell surface scFab library clone capable of binding to the CMV/HLA-A*02:01 SCT protein was measured for the yeast cell surface expression level through a mouse antibody 9e10 clone recognizing c-myc tag and an antibody recognizing Alexa488-conjugated mouse Fc, and the biotinylated antigen binding was measured using PE-conjugated streptavidin (streptavidin-R-phycoerythrin conjugate (SA-PE), Thermo), and thus clones having high scFab expression and high ability to bind to the biotinylated CMV/HLA-A*02:01 SCT protein were selected in the state in which the concentration of the biotinylated CMV/HLA-A*02:01 SCT protein was decreased. This procedure was repeated four times.

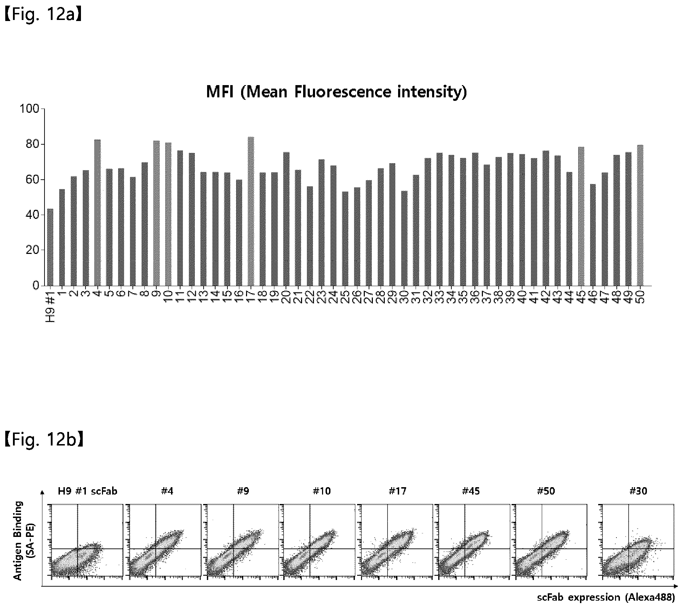

FIG. 12 shows the results of FACS analysis after binding of a total of 50 individual clones isolated from the library to 2 nM of the biotinylated CMV/HLA-A*02:01 SCT protein. When the #4, #9, #10, #17, #45, and #50 clones, having excellent scFab expression level and high antigen-binding strength, and the #30 clone, the graph pattern of which was slightly different from the remaining 49 clones, were selected and sequenced, all clones except for the #30 clone expressed scFab encoding the same amino acid sequence as #17.

Table 7 below shows the CDR amino acid sequences of the light-chain variable region and the heavy-chain variable region of the two converged clones. The changed amino acid sequence compared to the sequence of the H9 #1 clone is indicated in bold.

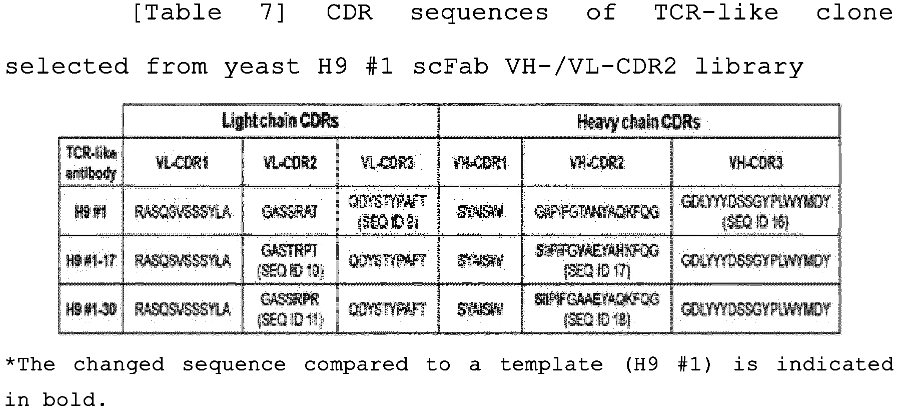

### Example 11: IgG conversion and identification of scFab clones having improved affinity to selected CMV/HLA-A*02:01 SCT protein

The heavy-chain variable region (VH) and light-chain variable region (VL) sequences of the selected H9 #1-17 and H9 #1-30 clones were introduced into the pcDNA3.4 vector encoding the heavy-chain constant region (CH1-hinge-CH2-CH3 domain) of mouse immunoglobulin 2a (mIgG2a) and the mouse kappa light-chain constant region (kappa domain), as in Example 5.

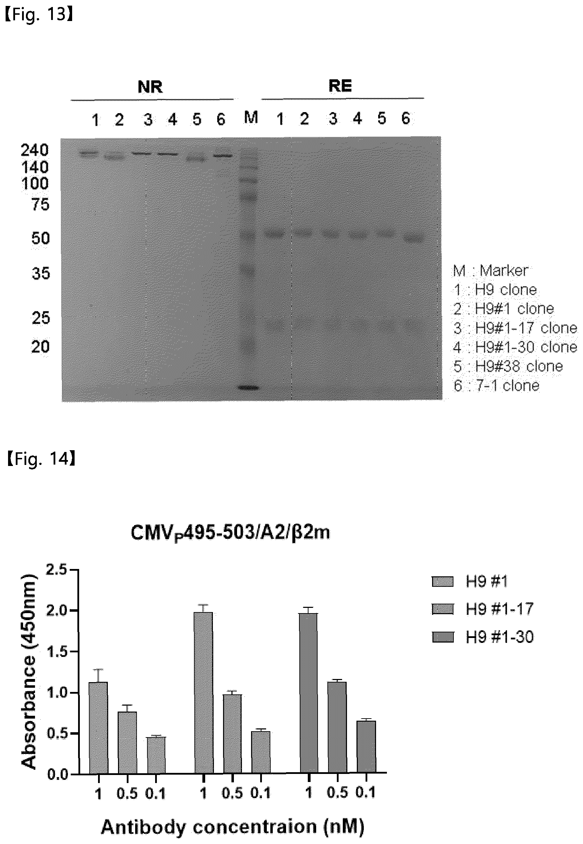

FIG. 13 shows the results of analysis of protein size and combination form through Coomassie Blue staining after H9 #1 and H9 #38 antibodies in which the affinity of the TCR-like antibody H9 was improved and H9 #1-17 and H9 #1-30 antibodies in which the affinity of H9 #1 was improved were expressed and purified in animal cells (HEK293F) and then 3 µg of protein was separated through 12% SDS-PAGE under reducing or non-reducing conditions. The H9 #1-17 and H9 #1-30 antibodies showed respective yields of 13.3 mg and 24.8 mg per l of HEK293F cell culture.

### Example 12: Evaluation of ability of selected TCR-like antibodies to bind to CMV/HLA-A*02:01 SCT protein

FIG. 14 shows the results of ELISA measurement to determine the ability of the H9 #1-17 and H9 #1-30 clones in IgG form expressed and purified in animal cells to bind to the CMV/HLA-A*02:01 SCT protein.

Specifically, EIA/RIA plates were coated with the CMV/HLA-A*02:01 SCT protein and the HPV/HLA-A*02:01 SCT protein at a concentration of 10 µg/ml in a PBS buffer at a pH of 7.4 at room temperature for 1 hour, and then washed three times with 0.05% PBST. After blocking at room temperature for 1 hour with a blocking buffer, the H9 #1, H9 #1-17, and H9 #1-30 antibodies were diluted to concentrations of 1 nM, 0.5 nM, and 0.1 nM in a blocking buffer, allowed to bind at room temperature for 1 hour, and then washed three times with a 0.05% PBST buffer. An HRP-conjugated anti-mouse Fc antibody as a labeling antibody was diluted 1:4000 in a blocking buffer and allowed to bind thereto. After reaction with a TMB ELISA solution at room temperature for 1 minute, the reaction was completed with H₂SO₄, and absorbance was measured at a wavelength of 450 nm.

Compared to H9 #1 in IgG form, the H9#1-17 and H9#1-30 clones in IgG form showed approximately double affinity to the CMV_{P}495-503/HLA-A*02:01 complex in ELISA.

### Example 13: Evaluation of ability of selected TCR-like antibodies to bind to cell-surface CMV_{P}495-503/HLA-A*02:01 complex

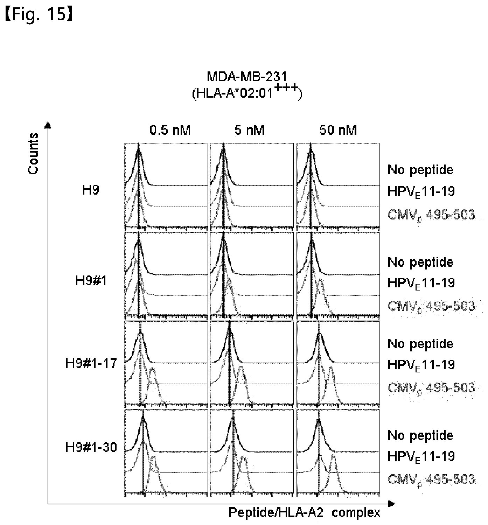

FIG. 15 shows the results of flow cytometry analysis to determine the ability of the H9 #1 antibody and the H9 #1-17 and H9 #1-30 antibodies having improved affinity to bind to the CMV_{P}495-503/HLA-A*02:01 complex, after pulsing of 4 µM CMVp495-503 peptide in an MDA-MB-231 breast cancer cell line expressing MHC-I (HLA-A*02:01). In order to confirm whether the antibodies having improved affinity are capable of effectively detecting the CMV_{P}495-503/HLA-A*02:01 complex that is expressed in a very low level on the cell surface, pulsing was carried out at a lower peptide concentration than in Examples 1 and 7. After completion of pulsing, the cells were washed with a FACS buffer and then centrifuged at 1300 rpm for 3 minutes to remove the supernatant. For each sample, 1.5×10⁵ cells were prepared, added with H9, H9 #1-17, and H9 #1-30 antibodies diluted to 0.5 nM, 5 nM, and 50 nM in 100 µl of a FACS buffer, and allowed to react at 4°C for 1 hour. After washing each sample with 1 ml of a FACS buffer, a F(ab')2 antibody that specifically recognizes Alexa647 (red fluorescence)-conjugated mouse Fc was diluted 1:600 in 100 µl of a FACS buffer, followed by reaction at 4°C for 30 minutes. Based on the results of flow cytometry analysis after washing with 1 ml of a FACS buffer, the experimental group treated with the HPV_{E}11-19 peptide did not show binding of TCR-like antibodies compared to the experimental group not treated with the peptide, and specific binding appeared only in the experimental group treated with the CMV_{P}495-503 peptide. Also, the H9 antibody did not detect the CMV_{P}495-503/HLA-A*02:01 complex even at the highest concentration of 50 nM, and compared to the H9 #1 antibody, the H9 #1-17 and H9 #1-30 antibodies detected a large amount of the CMV_{P}495-503/HLA-A*02:01 complex under the same treatment concentration conditions, indicating that the affinity thereof was improved. However, the H9 #1-17 and H9 #1-30 antibodies showed a phenomenon in which the peak of the graph was non-specifically shifted even in the experimental group not treated with the peptide, and the H9 #1-30 antibody, which showed this phenomenon more strongly, was not used in subsequent experiments.

### Example 14: Detection of cell-surface CMV_{P}495-503/HLA-A*02:01 complex using selected TCR-like antibody

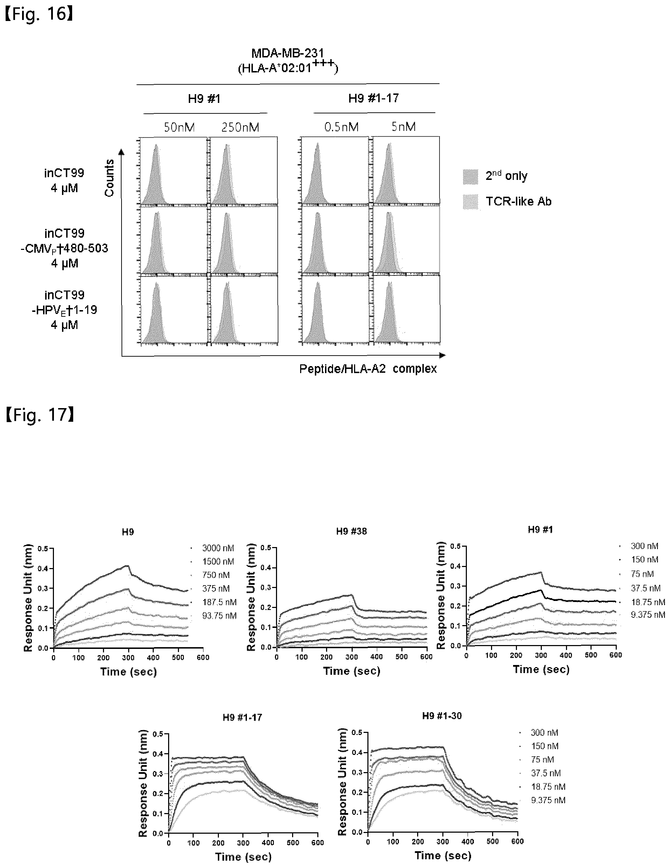

FIG. 16 shows the results of flow cytometry analysis to determine whether it was possible to induce formation of the cell-surface CMV_{P}495-503/HLA-A*02:01 complex upon treatment of an MDA-MB-231 cell line with the inCT99-CMV_{P}†480-503 fusion antibody, using the H9 #1-17 antibody.

Specifically, in a 24-well plate, an MDA-MB-231 cell line was diluted at a density of 1.8×10⁵ cells/ml per well in an RPMI medium containing 10% FBS and 1% ABAM antibiotic and cultured at 37°C and 5% CO₂ for 12 hours, after which the medium was removed, and inCT99, inCT99-CMV_{P}†480-503, and inCT99-HPV_{E}†1-19 fusion antibodies were diluted to a concentration of 4 µM in 1 ml of a medium, followed by culture at 37°C and 5% CO₂ for 18 hours. Thereafter, the cells were recovered by scraping and centrifuged at 1300 rpm for 3 minutes to remove the supernatant. The cells were washed with 1 ml of a FACS buffer and centrifuged to remove the supernatant. The cell pellets in each well were lysed with 100 µl of a FACS buffer and divided into two samples, one sample of which was used as a control containing only a secondary antibody. Each sample was added with the H9 #1 antibody at 50 nM and 250 nM and the H9 #1-17 antibody at 0.5 nM and 5 nM in 100 µl of a FACS buffer and then allowed to react at 4°C for 1 hour. After washing each sample with 1 ml of a FACS buffer, a F(ab')2 antibody that specifically recognizes Alexa647 (red fluorescence)-conjugated mouse Fc was diluted 1:600 in 100 µl of a FACS buffer, followed by reaction at 4°C for 30 minutes. Based on the results of flow cytometry analysis after washing with 1 ml of a FACS buffer, specific binding of the H9 #1 and H9 #1-17 antibodies was confirmed in the group treated with the inCT99-CMV_{P}†480-503 fusion antibody. In particular, the H9 #1-17 antibody was capable of effectively detecting the CMV_{P}495-503/HLA-A*02:01 complex even when used at 5 nM, which is a much lower concentration than the H9 #1 antibody, compared to the H9 #1 antibody, indicating that the affinity thereof was improved.

### Example 15: Analysis of binding affinity of TCR-like antibodies selected according to the present invention

FIG. 17 shows the results of analysis of the binding affinity of the TCR-like antibodies according to the present invention to the CMV_{P}495-503/HLA-A*02:01 complex.

Separately from the ELISA method, in order to quantitatively measure affinity to the CMV_{P}495-503/HLA-A*02:01 complex, affinity was measured according to the manufacturer's protocol using an Octet Qke (ForteBio, USA) instrument. The antibody was diluted to a concentration of 10 µg/ml in PBS, and for the H9 antibody, the CMV/HLA-A*02:01 SCT protein was serially diluted from 3 µM, and for the remaining clones, serial dilution was carried out from 300 nM, followed by addition of 200 µl thereof to an opaque 96-well plate through which light is incapable of passing. Antigen-binding kinetics of the antibody were analyzed depending on changes in the refractive index that occurs when the antigen is attached to the antibody and then detached therefrom while rapidly vibrating at 1000 rpm after moving the AMC (anti-mouse IgG Fc capture) sensor chip in the order of PBS solution, antibody, PBS solution, antigen, and PBS solution. The results thereof are shown in Table 8 below.

Thereby, the association rate constant (kₒₙ) of H9 #1-17 to the CMV/HLA-A*02:01 SCT protein was determined to be kₒₙ = 9.30×10⁵ 1/Ms, the dissociation rate constant (k_{off}) was determined to be k_{off} = 4.84×10⁻³ 1/s, and the equilibrium dissociation constant (K_{D}) was determined to be K_{D} = 5.20 nM. Based on these results, it was confirmed that the affinity thereof was improved by about 67 times compared to the existing H9 antibody reported in the literature, and by about 2.4 times compared to the H9 #1 antibody used as a library template for affinity improvement.

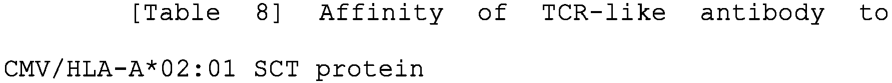

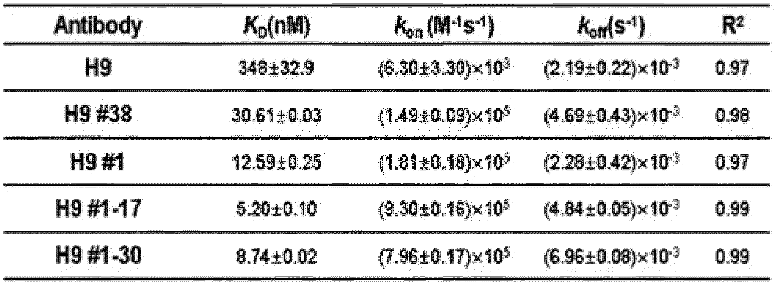

### [Industrial Applicability]

According to the present invention, a TCR-like antibody specific to a CMV_{P}495-503/HLA-A*02:01 complex has remarkably improved affinity compared to a conventional TCR-like antibody that specifically binds to the CMV_{P}495-503/HLA-A*02:01 complex.

In order to detect molecules having a very low expression level compared to other cell surface antigens, such as a peptide/MHC complex, high affinity thereto can generate great benefits. For example, in order to detect the CMV_{P}495-503/HLA-A*02:01 complex having a low expression level using a conventional antibody, treatment at high concentrations is required, and thus non-specific binding is observed. The TCR-like antibody having improved affinity according to the present invention is capable of detecting the cell-surface CMV_{P}495-503/HLA-A*02:01 complex even when used at low concentrations, and non-specific binding is not observed at high concentrations.

In addition, the cell-surface CMV_{P}495-503/HLA-A*02:01 complex can be detected even in cell lines in which the expression level of MHC molecules is relatively low, which cannot be detected with conventional antibodies, so the antibody of the present invention can be used for a wide variety of cell lines, and thus the usefulness thereof is high. Moreover, the construct having improved affinity to the CMV_{P}495-503/HLA-A*02:01 complex according to the present invention is capable of detecting the CMV_{P}495-503/HLA-A*02:01 complex even in an environment in which MHC loss occurs, such as a tumor microenvironment.

The TCR-like antibody specific to the CMV_{P}495-503/HLA-A*02:01 complex according to the present invention specifically binds to the CMV_{P}495-503/HLA-A*02:01 complex even at low concentrations, and does not non-specifically bind even at high concentrations, making it possible to produce a pharmaceutical composition for effective diagnosis and treatment of diseases caused by CMV infection.

In addition, the TCR-like antibody specific to the CMV_{P}495-503/HLA-A*02:01 complex according to the present invention can be developed as an effective cell therapeutic agent as a recombinant T-cell receptor.

### [Sequence List Free Text]

An electronic file is attached.

## Claims

1. A TCR (T-cell receptor)-like antibody specifically binding to an MHC-I complex presenting a viral protein antigen-derived peptide or an antigen-binding fragment thereof, comprising:
a heavy-chain CDR1 of SEQ ID NO: 12, a heavy-chain CDR2 selected from the group consisting of SEQ ID NOs: 13, 17, and 18, a heavy-chain CDR3 selected from the group consisting of SEQ ID NOs: 15 and 16, a light-chain CDR1 of SEQ ID NO: 5, a light-chain CDR2 selected from the group consisting of SEQ ID NOs: 6, 10, and 11, and a light-chain CDR3 selected from the group consisting of SEQ ID NOs: 8 and 9,
wherein the viral protein antigen-derived peptide is peptide 495-503 or peptide 480-503 of pp65 protein of human cytomegalovirus (CMV), and the MHC-I is HLA-A*02:01.

2. The antibody or the antigen-binding fragment thereof according to claim 1, comprising a heavy-chain variable region selected from the group consisting of SEQ ID NOs: 25 to 28.

3. The antibody or the antigen-binding fragment thereof according to claim 1, comprising a light-chain variable region selected from the group consisting of SEQ ID NOs: 20 to 23.

4. A nucleic acid encoding the antibody or the antigen-binding fragment thereof according to any one of claims 1 to 3.

5. An expression vector comprising the nucleic acid according to claim 4.

6. A isolated cell transformed with the expression vector according to claim 5.

7. A method of producing a TCR-like antibody or an antigen-binding fragment thereof, comprising:
(a) culturing the cell according to claim 6; and
(b) recovering an antibody or an antigen-binding fragment thereof from the cultured cell.

8. A composition comprising T cells expressing the antibody or the antigen-binding fragment thereof according to any one of claims 1 to 3 as a chimeric antigen receptor.

9. A composition for preventing or treating cancer or an infectious disease comprising the antibody or the antigen-binding fragment thereof according to any one of claims 1 to 3.

10. A composition for diagnosing cancer or an infectious disease comprising the antibody or the antigen-binding fragment thereof according to any one of claims 1 to 3.
